(19) Europäisches Patentamt
European Patent Office
Office européen des brevets

(11) EP 1 214 339 B1

(12) **EUROPEAN PATENT SPECIFICATION**

(45) Date of publication and mention of the grant of the patent: **01.08.2007 Bulletin 2007/31**

(21) Application number: **00975853.3**

(22) Date of filing: **14.09.2000**

(51) Int Cl.:
***C07K 14/22*** *(2006.01)* ***C07K 16/12*** *(2006.01)*
***C12N 1/21*** *(2006.01)* ***C12N 15/31*** *(2006.01)*
***A61K 38/16*** *(2006.01)* ***A61K 39/40*** *(2006.01)*
***G01N 33/569*** *(2006.01)*

(86) International application number: **PCT/EP2000/009034**

(87) International publication number: **WO 2001/019862 (22.03.2001 Gazette 2001/12)**

# (54) POLYPEPTIDES FROM MORAXELLA (BRANHAMELLA) CATARRHALIS

POLYPEPTIDE AUS MORAXELLA (BRANHAMELLA) CATARRHALIS

POLYPEPTIDES DE MORAXELLA (BRANHAMELLA) CATARRHALIS

(84) Designated Contracting States: **AT BE CH CY DE DK ES FI FR GB GR IE IT LI LU MC NL PT SE**

(30) Priority: **14.09.1999 GB 9921694**
**14.09.1999 GB 9921693**
**25.09.1999 GB 9922829**

(43) Date of publication of application: **19.06.2002 Bulletin 2002/25**

(73) Proprietor: **GlaxoSmithKline Biologicals s.a.**
**1330 Rixensart (BE)**

(72) Inventor: **Thonnard, Joelle,**
**GlaxoSmithKline Biologicals s.a.**
**B-1330 Rixensart (BE)**

(74) Representative: **Mallalieu, Catherine Louise et al**
**D Young & Co**
**120 Holborn**
**London EC1N 2DY (GB)**

(56) References cited:
**WO-A-97/41731** **WO-A-98/06851**

- **EMBL database, Heidelberg, FRG Trembl accession number Q99116 1 November 1996 MAIDEN, M.C.J. ET AL.: "MAJOR OUTER MEMBRANE PROTEIN P.IA (PROTEIN IA) (PIA) (FRAGMENT)" XP002162771 -& MAIDEN, M.C.J. ET AL.: "Comparison of the class 1 outer membrane proteins of eight serological reference strains of Neisseria meningitidis" MOLECULAR MICROBIOLOGY, vol. 5, no. 3, March 1991 (1991-03), pages 727-736, XP000926232**

EP 1 214 339 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### FIELD OF THE INVENTION

This invention relates to polynucleotides, (herein referred to as "BASB 129 polynucleotide(s)"), polypeptides encoded by them (referred to herein as "BASB 129" or "BASB129 polypeptide(s)"), recombinant materials and methods for their production. In another aspect, the invention relates to methods for using such polypeptides and polynucleotides, including vaccines against bacterial infections. In a further aspect, the invention relates to diagnostic assays for detecting infection of certain pathogens.

### BACKGROUND OF THE INVENTION

*Moraxella catarrhalis* (also named *Branhamella catarrhalis)* is a Gram negative bacteria frequently isolated from the human upper respiratory tract. It is responsible for several pathologies the main ones being otitis media in infants and children, and pneumonia in elderlies. It is also responsible of sinusitis, nosocomial infections and less frequently of invasive diseases.

Otitis media is an important childhood disease both by the number of cases and its potential sequelae. More than 3.5 millions cases are recorded every year in the United States, and it is estimated that 80 % of the children have experienced at least one episode of otitis before reaching the age of 3 (Klein, JO (1994) Clin.Inf.Dis 19:823). Left untreated, or becoming chronic, this disease may lead to hearing losses that could be temporary (in the case of fluid accumulation in the middle ear) or permanent (if the auditive nerve is damaged). In infants, such hearing losses may be responsible for a delayed speech learning.

Three bacterial species are primarily isolated from the middle ear of children with otitis media: *Streptococcus pneumoniae,* non typeable *Haemophilus influenza* (NTHi) and *M. catarrhalis.* They are present in 60 to 90 % of the cases. A review of recent studies shows that *S. pneumoniae* and NTHi represent both about 30 %, and *M catarrhalis* about 15 % of the otitis media cases (Murphy, TF (1996) Microbiol.Rev. 60:267). Other bacteria could be isolated from the middle ear *(H. influenza* type B, *S. pyogenes* etc) but at a much lower frequency (2 % of the cases or less).

Epidemiological data indicate that, for the pathogens found in the middle ear, the colonization of the upper respiratory tract is an absolute prerequisite for the development of an otitis; other are however also required to lead to the disease (Dickinson, DP et al. (1988) J. Infect.Dis. 158:205, Faden, HL et al. (1991) Ann.Otorhinol.Laryngol. 100: 612). These are important to trigger the migration of the bacteria into the middle ear via the Eustachian tubes, followed by the initiation of an inflammatory process. These factors are unknown todate. It has been postulated that a transient anomaly of the immune system following a viral infection, for example, could cause an inability to control the colonization of the respiratory tract (Faden, HL et al (1994) J. Infect.Dis. 169:1312). An alternative explanation is that the exposure to environmental factors allow a more important colonization of some children, who subsequently become susceptible to the development of otitis media because of the sustained presence of middle ear pathogens (Murphy, TF (1996) Microbiol.Rev. 60:267).

The immune response to *M. catarrhalis* is poorly characterized. The analysis of strains isolated sequentially from the nasopharynx of babies followed from 0 to 2 years of age, indicates that they get and eliminate frequently new strains. This indicates that an efficacious immune response against this bacteria is mounted by the colonized children (Faden, HL et al (1994) J. Infect.Dis. 169:1312).

In most adults tested, bactericidal antibodies have been identified (Chapman, AJ et al. (1985) J. Infect.Dis. 151:878). Strains of *M. catarrhalis* present variations in their capacity to resist serum bactericidal activity: in general, isolates from diseased individuals are more resistant than those who are simply colonized (Hol, C et al. (1993) Lancet 341:1281, Jordan, KL et al. (1990) Am.J.Med. 88 (suppl. 5A):28S). Serum resistance could therfore be considered as a virulence factor of the bacteria. An opsonizing activity has been observed in the sera of children recovering from otitis media.

The antigens targetted by these different immune responses in humans have not been identified, with the exception of OMP B1, a 84 kDa protein which expression is regulated by iron, and that is recognized by the sera of patients with pneumonia (Sethi, S, et al. (1995) Infect.Immun. 63:1516), and of UspA 1 and UspA2 (Chen D. et al.(1999), Infect.Immun. 67:1310).

A few other membrane proteins present on the surface of *M catarrhalis* have been characterized using biochemical method, or for their potential implication in the induction of a protective immunity (for review, see Murphy, TF (1996) Microbiol.Rev. 60:267). In a mouse pneumonia model, the presence of antibodies raised against some of them (UspA, CopB) favors a faster clearance of the pulmonary infection. Another polypeptide (OMP CD) is highly conserved among *M. catarrhalis* strains, and presents homologies with a porin of *Pseudomonas aeruginosa,* which, has been demonstrated efficacious against this bacterium in animal models.

WO 00/78968 describes nucleotide sequences and identifies open reading frames from the genome of *Moraxella*

*catarrhalis.*

The frequency of *Moraxella catarrhalis* infections has risen dramatically in the past few decades. This has been attributed to the emergence of multiply antibiotic resistant strains and an increasing population of people with weakened immune systems. It is no longer uncommon to isolate *Moraxella catarrhalis* strains that are resistant to some or all of the standard antibiotics. This phenomenon has created an unmet medical need and demand for new anti-microbial agents, vaccines, drug screening methods, and diagnostic tests for this organism.

## SUMMARY OF THE INVENTION

The present invention relates to BASB 129, in particular BASB 129 polypeptides and BASB 129 polynucleotides, recombinant materials and methods for their production. We describe methods for using such polypeptides and polynucleotides, including prevention and treatment of microbial diseases, amongst others. We also describe diagnostic assays for detecting diseases associated with microbial infections and conditions associated with such infections, such as assays for detecting expression or activity of BASB 129 polynucleotides or polypeptides.

According to a first aspect of the present invention there is provided an immunogenic fragment of an isolated polypeptide comprising an amino acid sequence which has at least 85% identity to the amino acid sequence of: SEQ ID NO:2 over the entire length of SEQ ID NO:2 in which the immunogenic fragment comprises at least 15 contiguous amino acids from the amino acid sequence of SEQ ID NO:2 and which is capable of raising an immune response if necessary, when coupled to a carrier, which recognises the polypeptide of SEQ ID NO:2, and wherein the immunogenic fragment is not the amino acid sequence of SEQ ID NO:2.

According to another aspect of the present invention there is provided an isolated polynucleotide encoding the immunogenic fragment of the invention.

According to another aspect of the present invention there is provided an expression vector comprising the isolated polynucleotide of the invention.

According to another aspect of the present invention there is provided a recombinant live microorganism comprising the expression vector of the invention.

According to another aspect of the present invention there is provided a host cell comprising the expression vector of the invention.

According to another aspect of the present invention there is provided an isolated subcellular fraction or an isolated membrane of the host cell of the invention.

According to another aspect of the present invention there is provided a membrane of a recombinant microorganism which has upregulated expression of an isolated polypeptide comprising an amino acid sequence which has at least 85% identity to the amino acid sequence of: SEQ ID NO:2 over the entire length of SEQ ID NO:2, or the immunogenic fragment of the present invention.

According to another aspect of the present invention there is provided a process for producing the immunogenic fragment of the invention comprising culturing a host cell of the invention under conditions sufficient for the production of said polypeptide and recovering the polypeptide from the culture medium.

According to another aspect of the present invention there is provided a process for expressing a polynucleotide of the invention comprising transforming a host cell with an expression vector comprising at least one of said polynucleotides and culturing said host cell under conditions sufficient for expression of any one of said polynucleotides.

According to another aspect of the present invention there is provided a bacterial outer-membrane vesicle obtained from a host cell comprising a nucleic acid vector comprising a nucleotide sequence encoding a polypeptide that has at least 85% identity to the amino acid sequence of SEQ ID NO:2 over the entire length of SEQ ID NO:2; a nucleotide sequence that has at least 83% identity to a nucleotide sequence encoding a polypeptide of SEQ ID NO:2 over the entire coding region; a nucleotide sequence which has at least 85% identity to that of SEQ ID NO:1 over the entire length of SEQ ID NO:1; or a polynucleotide encoding an immunogenic fragment of the invention having a modified upstream region containing a heterologous regulatory element of the present invention having a modified upstream region containing a heterologous element.

According to another aspect of the present invention there is provided a vaccine composition comprising an effective amount of an isolated polypeptide comprising an amino acid sequence which has at least 85% identity to the amino acid sequence of: SEQ ID NO:2 over the entire length of SEQ ID NO:2, or the immunogenic fragment of the present invention and a pharmaceutically acceptable carrier.

According to another aspect of the present invention there is provided a vaccine composition comprising an effective amount of the bacterial outer-membrane vesicle of the invention and a pharmaceutically acceptable carrier.

According to another aspect of the present invention there is provided an antibody generated against the immunological fragment of the invention.

According to another aspect of the present invention there is provided a method of diagnosing a *Moraxella catarrhalis* infection, comprising identifying an immunogenic fragment or an antibody of the invention, present within a biological sample from an animal suspected of having such an infection.

According to another aspect of the present invention there is provided use of a composition comprising an immunologically effective amount of an isolated polypeptide comprising an amino acid sequence which has at least 85% identity to the amino acid sequence of: SEQ ID NO:2 over the entire length of SEQ ID NO:2, or an immunogenic fragment of the present invention in the preparation of a medicament for use in generating an immune response in an animal.

According to another aspect of the present invention there is provided use of a composition comprising an immunologically effective amount of a polynucleotide comprising a nucleotide sequence encoding a polypeptide that has at least 85% identity to the amino acid sequence of SEQ ID NO:2 over the entire length of SEQ ID NO:2 or a nucleotide sequence complementary to said isolated polynucleotide; a polynucleotide comprising a nucleotide sequence that has at least 85% identity to a nucleotide sequence encoding a polypeptide of SEQ ID NO:2 over the entire coding region or a nucleotide sequence complementary to said isolated polynucleotide; a polynucleotide which comprises a nucleotide sequence which has at least 85% identity to that of SEQ ID NO:1 over the entire length of SEQ ID NO: or a nucleotide sequence complementary to said isolated polynucleotide; or a polynucleotide of the invention in the preparation of a medicament for use in generating an immune response in an animal.

According to another aspect of the present invention there is provided a therapeutic composition useful in treating humans with *Moraxella catarrhalis* disease comprising at least one antibody of the invention and a suitable pharmaceutical carrier.

## DESCRIPTION OF THE INVENTION

There is disclosed BASB129 polypeptides and polynucleotides as described in greater detail below. In particular, there is described polypeptides and polynucleotides of BASB129 of *Moraxella catarrhalis,* which is related by amino acid sequence homology to *Haemophilus influenzae* major outer membrane protein P2. There is also described BASB 129 having the nucleotide and amino acid sequences set out in SEQ ID NO:1 and SEQ ID NO:2 respectively. It is understood that sequences recited in the Sequence Listing below as "DNA" represent an exemplification of one embodiment of the invention, since those of ordinary skill will recognize that such sequences can be usefully employed in polynucleotides in general, including ribopolynucleotides.

### Polypeptides

There is desribed polypeptides of *Moraxella catarrhalis* referred to herein as "BASB 129" and "BASB 129 polypeptides" as well as biologically, diagnostically, prophylactically, clinically or therapeutically useful variants thereof, and compositions comprising the same.

There is further described:

(a) an isolated polypeptide which comprises an amino acid sequence which has at least 85% identity, preferably at least 90% identity, more preferably at least 95% identity, most preferably at least 97-99% or exact identity, to that of SEQ ID N0:2;

(b) a polypeptide encoded by an isolated polynucleotide comprising a polynucleotide sequence which has at least 85% identity, preferably at least 90% identity, more preferably at least 95% identity, even more preferably at least 97-99% or exact identity to SEQ ID NO:1 over the entire length of SEQ ID NO:1; or

(c) a polypeptide encoded by an isolated polynucleotide comprising a polynucleotide sequence encoding a polypeptide which has at least 85% identity, preferably at least 90% identity, more preferably at least 95% identity, even more preferably at least 97-99% or exact identity, to the amino acid sequence of SEQ ID NO:2.

The BASB 129 polypeptide provided in SEQ ID NO:2 is the BASB 129 polypeptide from *Moraxella catarrhalis* strain Mc2931 (ATCC 43617).

The invention also provides an immunogenic fragment of a BASB 129 polypeptide, that is, a contiguous portion of the BASB 129 polypeptide which has the same or substantially the same immunogenic activity as the polypeptide comprising the amino acid sequence of SEQ ID NO:2; That is to say, the fragment (if necessary when coupled to a carrier) is capable of raising an immune response which recognises the BASB 129 polypeptide. Such an immunogenic fragment may include, for example, the BASB 129 polypeptide lacking an N-terminal leader sequence, and/or a transmembrane domain and/or a C-terminal anchor domain. In a preferred aspect the immunogenic fragment of BASB 129 according to the invention comprises substantially all of the extracellular domain of a polypeptide which has at least 85% identity, preferably at least 90% identity, more preferably at least 95% identity, most preferably at least 97-99% identity, to that of SEQ ID NO:2 over the entire length of SEQ ID NO:2.

A fragment is a polypeptide having an amino acid sequence that is entirely the same as part but not all of any amino acid sequence of any polypeptide described. As with BASB129 polypeptides, fragments may be "free-standing," or comprised within a larger polypeptide of which they form a part or region, most preferably as a single continuous region in a single larger polypeptide.

Preferred fragments include, for example, truncation polypeptides having a portion of an amino acid sequence of SEQ ID NO:2 or of variants thereof, such as a continuous series of residues that includes an amino- and/or carboxyl-terminal amino acid sequence. Degradation forms of the polypeptides of the invention produced by or in a host cell, are also preferred. Further preferred are fragments characterized by structural or functional attributes such as fragments that comprise alpha-helix and alpha-helix forming regions, beta-sheet and beta-sheet-forming regions, turn and tum-forming regions, coil and coil-forming regions, hydrophilic regions, hydrophobic regions, alpha amphipathic regions, beta amphipathic regions, flexible regions, surface-forming regions, substrate binding region, and high antigenic index regions.

Further preferred fragments include an isolated polypeptide comprising an amino acid sequence having at least 15, 20, 30, 40, 50 or 100 contiguous amino acids from the amino acid sequence of SEQ ID NO:2, or an isolated polypeptide comprising an amino acid sequence having at least 15, 20, 30, 40, 50 or 100 contiguous amino acids truncated or deleted from the amino acid sequence of SEQ ID NO:2.

Fragments of the invention may be employed for producing the corresponding full-length polypeptide by peptide synthesis; therefore, these fragments may be employed as intermediates for producing the full-length polypeptides of the invention.

Particularly preferred are variants in which several, 5-10, 1-5, 1-3, 1-2 or 1 amino acids are substituted, deleted, or added in any combination

The polypeptides, or immunogenic fragments, of the invention may be in the form of the "mature" protein or may be a part of a larger protein such as a precursor or a fusion protein. It is often advantageous to include an additional amino acid sequence which contains secretory or leader sequences, pro-sequences, sequences which aid in purification such as multiple histidine residues, or an additional sequence for stability during recombinant production. Furthermore, addition of exogenous polypeptide or lipid tail or polynucleotide sequences to increase the immunogenic potential of the final molecule is also considered.

There is also described genetically engineered soluble fusion proteins comprising the disclosed polypeptide, or a fragment thereof, and various portions of the constant regions of heavy or light chains of immunoglobulins of various subclasses (IgG, IgM, IgA, IgE). Preferred as an immunoglobulin is the constant part of the heavy chain of human IgG, particularly IgG 1, where fusion takes place at the hinge region. In a particular embodiment, the Fc part can be removed simply by incorporation of a cleavage sequence which can be cleaved with blood clotting factor Xa.

Furthermore, there is described processes for the preparation of these fusion proteins by genetic engineering, and to the use thereof for drug screening, diagnosis and therapy. There is also described polynucleotides encoding such fusion proteins. Examples of fusion protein technology can be found in International Patent Application Nos. WO94/29458 and WO94/22914.

The proteins may be chemically conjugated, or expressed as recombinant fusion proteins allowing increased levels to be produced in an expression system as compared to non-fused protein. The fusion partner may assist in providing T helper epitopes (immunological fusion partner), preferably T helper epitopes recognised by humans, or assist in expressing the protein (expression enhancer) at higher yields than the native recombinant protein. Preferably the fusion partner will be both ammunological fusion partner and expression enhancing partner.

Fusion partners include protein D from *Haemophilus influenzae* and the non-structural protein from influenzae virus, NS1 (hemagglutinin). Another fusion partner is the protein known as LytA. Preferably the C terminal portion of the molecule is used. Lyta is derived from *Streptococcus pneumoniae* which synthesize an N-acetyl-L-alanine amidase, amidase LytA, (coded by the lytA gene {Gene, 43 (1986) page 265-272}) an autolysin that specifically degrades certain bonds in the peptidoglycan backbone. The C-terminal domain of the LytA protein is responsible for the affinity to the choline or to some choline analogues such as DEAE. This property has been exploited for the development of E.coli C-LytA expressing plasmids useful for expression of fusion proteins. Purification of hybrid proteins containing the C-LytA fragment at its amino terminus has been described {Biotechnology: 10, (1992) page 795-798}. It is possible to use the repeat portion of the LytA molecule found in the C terminal end starting at residue 178, for example residues 188 - 305.

There is also described variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr.

The disclosed polypeptides can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

It is most preferred that the polypeptide is derived from *Moraxella catarrhalis,* however, it may preferably be obtained from other organisms of the same taxonomic genus. The polypeptide may also be obtained, for example, from organisms of the same taxonomic family or order.

**Polynucleotides**

There is described polynucleotides that encode BASB 129 polypeptides, particularly polynucleotides that encode the polypeptide herein designated BASB 129.

We describe polynucleotides comprising a region encoding BASH 129 polypeptides comprising a sequence set out in SEQ ID NO:1 which includes a full length gene, or a variant thereof.

The BASB 129 polynucleotide provided in SEQ ID NO:1 is the BASB 129 polynucleotides from *Moraxella catarrhalis* strain Mc2931 (ATCC 43617).

There is described an isolated nucleic acid molecules encoding and/or expressing BASB129 polypeptides and polynucleotides, particularly *Moraxella catarrhalis* BASB129 polypeptides and polynucleotides, including, for example, unprocessed RNAs, ribozyme RNAs, mRNAs, cDNAs, genomic DNAs, B- and Z-DNAs. Further embodiments of the invention include biologically, diagnostically, prophylactically, clinically or therapeutically useful polynucleotides and polypeptides, and variants thereof, and compositions comprising the same.

There is also described isolated polynucleotides, including at least one full length gene, that encodes a BASB 129 polypeptide having a deduced amino acid sequence of SEQ ID NO:2 and polynucleotides closely related thereto and variants thereof.

There is also described a BASB 129 polypeptide from *Moraxella catarrhalis* comprising or consisting of an amino acid sequence of SEQ ID NO:2 or a variant thereof.

Using the information provided herein, such as a polynucleotide sequence set out in SEQ ID NO:1, a polynucleotide encoding BASB129 polypeptide may be obtained using standard cloning and screening methods, such as those for cloning and sequencing chromosomal DNA fragments from bacteria using *Moraxella catarrhalis* Catlin cells as starting material, followed by obtaining a full length clone. For example, to obtain a polynucleotide sequence, such as a polynucleotide sequence given in SEQ ID NO:1, typically a library of clones of chromosomal DNA of *Moraxella catarrhalis* Catlin in *E. coli* or some other suitable host is probed with a radiolabeled oligonucleotide, preferably a 17-mer or longer, derived from a partial sequence. Clones carrying DNA identical to that of the probe can then be distinguished using stringent hybridization conditions. By sequencing the individual clones thus identified by hybridization with sequencing primers designed from the original polypeptide or polynucleotide sequence it is then possible to extend the polynucleotide sequence in both directions to determine a full length gene sequence. Conveniently, such sequencing is performed, for-example, using denatured double stranded DNA prepared from a plasmid clone. Suitable techniques are described by Maniatis, T., Fritsch, E.F. and Sambrook et al., MOLECULAR CLONING, A LABORATORY MANUAL, 2nd Ed.; Cold Spring Harbor Laboratory Press, Cold Spring Harbor, New York (1989). (see in particular Screening By Hybridization 1.90 and Sequencing Denatured Double-Stranded DNA Templates 13.70). Direct genomic DNA sequencing may also be performed to obtain a full length gene sequence. Illustrative of the invention, each polynucleotide set out in SEQ ID NO: 1 was discovered in a DNA library derived from *Moraxella catarrhalis.*

Moreover, the DNA sequence set out in SEQ ID NO:1 contains an open reading frame encoding a protein having about the number of amino acid residues set forth in SEQ ID NO:2 with a deduced molecular weight that can be calculated using amino acid residue molecular weight values well known to those skilled in the art.

The polynucleotide of SEQ ID NO:1, between the start codon at nucleotide number 1 and the stop codon which begins at nucleotide number 1033 of SEQ ID NO:1, encodes the polypeptide of SEQ ID NO:2.

There is described an isolated polynucleotide comprising or consisting of:

(a) a polynucleotide sequence which has at least 85% identity, preferably at least 90% identity, more preferably at least 95% identity, even more preferably at least 97-99% or exact identity to SEQ ID NO: 1 over the entire length of SEQ ID NO:1; or

(b) a polynucleotide sequence encoding a polypeptide which has at least 85% identity, preferably at least 90% identity, more preferably at least 95% identity, even more preferably at least 97-99% or 100% exact, to the amino acid sequence of SEQ ID NO:2, over the entire length of SEQ ID NO:2.

A polynucleotide encoding a polypeptide of the present invention, including homologs and orthologs from species other than *Moraxella catarrhalis,* may be obtained by a process which comprises the steps of screening an appropriate library under stringent hybridization conditions (for example, using a temperature in the range of 45 - 65°C and an SDS concentration from 0.1-1%) with a labeled or detectable probe consisting of or comprising the sequence of SEQ ID NO:1 or a fragment thereof; and isolating a full-length gene and/or genomic clones containing said polynucleotide sequence.

There is described a polynucleotide sequence identical over its entire length to a coding sequence (open reading frame) in SEQ ID NO:1. There is also described a coding sequence for a mature polypeptide or a fragment thereof, by itself as well as a coding sequence for a mature polypeptide or a fragment in reading frame with another coding sequence, such as a sequence encoding a leader or secretory sequence, a pre-, or pro- or prepra-protein sequence. The described polynucleotide may also contain at least one non-coding sequence, including for example, but not limited to at least one non-coding 5' and 3' sequence, such as the transcribed but non-translated sequences, termination signals (such as rho-dependent and rho-independent termination signals), ribosome binding sites, Kozak sequences, sequences that stabilize mRNA, introns, and polyadenylation signals. The polynucleotide sequence may also comprise additional coding sequence encoding additional amino acids. For example, a marker sequence that facilitates purification of the fused polypeptide can be encoded. In certain embodiments of the invention, the marker sequence is a hexa-histidine peptide, as provided in the pQE vector (Qiagen, Inc.) and described in Gentz et al., Proc. Natl. Acad. Sci., USA 86: 821-824 (1989), or an HA peptide tag (Wilson et al., Cell 37: 767 (1984), both of which may be useful in purifying polypeptide sequence fused to them. Polynucleotides of the invention also include, but are not limited to, polynucleotides comprising a structural gene and its naturally associated sequences that control gene expression.

The nucleotide sequence encoding BASB 129 polypeptide of SEQ ID NO:2 may be identical to the polypeptide encoding sequence contained in nucleotides 1 to 1033 of SEQ ID NO: 1. Alternatively the nucleotide sequence encoding BASB 129 may be a sequence, which as a result of the redundancy (degeneracy) of the genetic code, also encodes the polypeptide of SEQ ID NO:2.

The term "polynucleotide encoding a polypeptide" as used herein encompasses polynucleotides that include a sequence encoding a polypeptide herein described, particularly a bacterial polypeptide and more particularly a polypeptide of the *Moraxella catarrhalis* BASB129 having an amino acid sequence set out in SEQ ID NO:2. The term also encompasses polynucleotides that include a single continuous region or discontinuous regions encoding the polypeptide (for example, polynucleotides interrupted by integrated phage, an integrated insertion sequence, an integrated vector sequence, an integrated transposon sequence, or due to RNA editing or genomic DNA reorganization) together with additional regions; that also may contain coding and/or non coding sequences.

There is also described variants of the polynucleotides disclosed herein that encode variants of a polypeptide having a deduced amino acid sequence of SEQ ID NO:2.

Fragments of the disclosed polynucleotides may be used, for example, to synthesize full-length polynucleotides.

There is also described polynucleotides encoding BASB129 variants, that have the amino acid sequence of BASB 129 polypeptide of SEQ ID NO:2 in which several, a few, 5 to 10, 1 to 5, 1 to 3, 2, 1 or no amino acid residues are substituted, modified, deleted and/or added, in any combination. Especially preferred among these are silent substitutions, additions and deletions, that do not alter the properties and activities of BASB 129 polypeptide.

There is also described polynucleotides that are at least 85% identical over their entire length to a polynucleotide encoding BASB 129 polypeptide having an amino acid sequence set out in SEQ ID NO:2, and polynucleotides that are complementary to such polynucleotides. Alternatively, most highly preferred are polynucleotides that comprise a region that is at least 90% identical over its entire length to a polynucleotide encoding BASB 129 polypeptide and polynucleotides complementary thereto. In this regard, polynucleotides at least 95% identical over their entire length to the same are particularly preferred. Furthermore, those with at least 97% are highly preferred among those with at least 95%, and among these those with at least 98% and at least 99% are particularly highly preferred, with at least 99% being the more preferred.

There is also described polynucleotides encoding polypeptides that retain substantially the same biological function or activity as the mature polypeptide encoded by a DNA of SEQ ID N0:1.

There is also described polynucleotides that hybridize, particularly under stringent conditions, to BASB 129 polynucleotide sequences, such as the polynucleotide in SEQ ID NO:1. There is also described polynucleotides that hybridize to the polynucleotide sequences provided herein. In this regard, polynucleotides are described that hybridize under stringent conditions to the polynucleotides described herein. As herein used, the terms "stringent conditions" and "stringent hybridization conditions" mean hybridization occurring only if there is at least 95% and preferably at least 97% identity between the sequences. A specific example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml of denatured, sheared salmon sperm DNA, followed by washing the hybridization support in 0.1 x SSC at about 65°C. Hybridization and wash conditions are well known and exemplified in Sambrook, et al., Molecular Cloning: A Laboratory Manual, Second Edition, Cold Spring Harbor, N.Y., (1989), particularly Chapter 1 I therein. Solution hybridization may also be used with the polynucleotide sequences provided by the invention.

There is also described a polynucleotide consisting of or comprising a polynucleotide sequence obtained by screening an appropriate library containing the complete gene for a polynucleotide sequence set forth in SEQ ID NO:1 under stringent hybridization conditions with a probe having the sequence of said polynucleotide sequence set forth in SEQ ID NO: 1 or a fragment thereof; and isolating said polynucleotide sequence. Fragments useful for obtaining such a polynucleotide include, for example, probes and primers fully described elsewhere herein.

As discussed elsewhere herein regarding polynucleotide assays, for instance, the disclosed polynucleotides may be used as a hybridization probe for RNA, cDNA and genomic DNA to isolate full-length cDNAs and genomic clones encoding BASB129 and to isolate cDNA and genomic clones of other genes that have a high identity particularly high sequence identity, to the BASB129 gene. Such probes generally will comprise at least 15 nucleotide residues or base pairs. Preferably, such probes will have at least 30 nucleotide residues or base pairs and may have at least 50 nucleotide residues or base pairs. Particularly preferred probes will have at least 20 nucleotide residues or base pairs and will have less than 30 nucleotide residues or base pairs.

A coding region of a BASB 129 gene may be isolated by screening using a DNA sequence provided in SEQ ID NO: to synthesize an oligonucleotide probe. A labeled oligonucleotide having a sequence complementary to that of a gene of the invention is then used to screen a library of cDNA, genomic DNA or mRNA to determine which members of the library the probe hybridizes to.

There are several methods available and well known to those skilled in the art to obtain full-length DNAs, or extend short DNAs, for example those based on the method of Rapid Amplification of cDNA ends (RACE) (see, for example, Frohman, et al., PNAS USA 85: 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end. Nucleic acid amplification (PCR) is then carried out to amplify the "missing" 5' end of the DNA using a combination of gene specific and adaptor specific oligonucleotide primers. The PCR reaction is then repeated using "nested" primers, that is, primers designed to anneal within the amplified product (typically an adaptor specific primer that anneals further 3' in the adaptor sequence and a gene specific primer that anneals further 5' in the selected gene sequence). The products of this reaction can then be analyzed by DNA sequencing and a full-length DNA constructed either by joining the product directly to the existing DNA to give a complete sequence, or carrying out a separate full-length PCR using the new sequence information for the design of the 5' primer.

The disclosed polynucleotides and polypeptides may be employed; for example; as research reagents and materials for discovery of treatments of and diagnostics for diseases, particularly human diseases, as further discussed herein relating to polynucleotide assays.

The polynucleotides that are oligonucleotides derived from a sequence of SEQ ID NO:1 may be used in the processes herein as described, but preferably for PCR, to determine whether or not the polynucleotides identified herein in whole or in part are transcribed in bacteria in infected tissue. It is recognized that such sequences will also have utility in diagnosis of the stage of infection and type of infection the pathogen has attained.

There is also described polynucleotides that encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

For each and every polynucleotide described herein there is a polynucleotide complementary to it. It is preferred that these complementary polynucleotides are fully complementary to each polynucleotide with which they are complementary.

A precursor protein, having a mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In addition to the standard A, G, C, T/U representations for nucleotides, the term "N" may also be used in describing certain polynucleotides of the invention. "N" means that any of the four DNA or RNA nucleotides may appear at such a designated position in the DNA or RNA sequence, except it is preferred that N is not a nucleic acid that when taken in combination with adjacent nucleotide positions, when read in the correct reading frame, would have the effect of generating a premature termination codon in such reading frame.

In sum, a polynucleotide described herein may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide.

There is described the use of the disclosed polynucleotide for therapeutic or prophylactic purposes, in particular genetic immunization.

The use of the disclosed polynucleotide in genetic immunization will preferably employ a suitable delivery method such as direct injection of plasmid DNA into muscles (Wolff et al., Hum Mol Genet (1992) 1: 363, Manthorpe et al., Hum. Gene Ther. (1983) 4: 419), delivery of DNA complexed with specific protein carriers (Wu et al., J Biol Chem. (1989) 264: 16985), coprecipitation of DNA with calcium phosphate (Benvenisty & Reshef, PNAS USA, (1986) 83: 9551), encapsulation of DNA in various forms of liposomes (Kaneda et al., Science (1989) 243: 375), particle bombardment (Tang et al., Nature (1992) 356:152, Eisenbraun et al., DNA Cell Biol (1993) 12: 791) and *in vivo* infection using cloned retroviral vectors (Seeger et al., PNAS USA (1984) 81: 5849).

**Vectors, Host Cells, Expression Systems**

There is also described vectors that comprise the disclosed polynucleotide or polynucleotides, host-cells that are genetically engineered with these vectors and the production of the disclosed polypeptides by recombinant techniques. Cell-free translation systems can also be employed to produce such proteins using RNAs derived from the described DNA constructs.

The recombinant polypeptides disclosed herein may be prepared by processes well known in those skilled in the art from genetically engineered host cells comprising expression systems. There is also described expression systems that comprise the disclosed polynucleotide or polynucleotides, host cells which are genetically engineered with such expression systems, and the production of the disclosed polypeptides by recombinant techniques.

For recombinant production of the disclosed polypeptides, host cells can be genetically engineered to incorporate expression systems or portions thereof or the disclosed polynucleotides. Introduction of a polynucleotide into the host cell can be effected by methods described in many standard laboratory manuals, such as Davis, et al., BASIC METHODS IN MOLECULAR BIOLOGY, (1986) and Sambrook, et al., MOLECULAR CLONING: A LABORATORY MANUAL, 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as cells of streptococci, staphylococci, enterococci, *E. coli,* streptomyces, cyanobacteria, *Bacillus subtilis, Neisseria mertingitidis* and *Moraxella catarrhalis;* fungal cells, such as cells of a yeast, *Kluveromyces, Saccharomyces,* a basidiomycete, *Candida albicans* and *Aspergillus;* insect cells such as cells of *Drosophila* S2 and *Spodoptera* Sf9; animal cells such as CHO, COS, HeLa, C127, 3T3, BHK, 293, CV-1 and Bowes melanoma cells; and plant cells, such as cells of a gymnosperm or angiosperm.

A great variety of expression systems can be used to produce the disclosed polypeptides. Such vectors include, among others, chromosomal-, episomal- and virus-derived vectors, for example, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, picornaviruses, retroviruses, and alphaviruses and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al., MOLECULAR CLONING, A LABORATORY MANUAL, (supra).*

In recombinant expression systems in eukaryotes, for secretion of a translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

The disclosed polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, ion metal affinity chromatography (IMAC) is employed for purification. Well known techniques for refolding proteins may be employed to regenerate active conformation when the polypeptide is denatured during intracellular synthesis, isolation and or purification.

The expression system may also be a recombinant live microorganism, such as a virus or bacterium. The gene of interest can be inserted into the genome of a live recombinant virus or bacterium. Inoculation and *in vivo* infection with this live vector will lead to *in vivo* expression of the antigen and induction of immune responses. Viruses and bacteria used for this purpose are for instance: poxviruses (e.g; vaccinia, fowlpox, canarypox), alphaviruses (Sindbis virus, Semliki Forest Virus, Venezuelian Equine Encephalitis Virus), adenoviruses, adeno-associated virus, picornaviruses (poliovirus, rhinovirus), herpesviruses (varicella zoster virus, etc), Listeria, Salmonella , Shigella, BCG. These viruses and bacteria can be virulent, or attenuated in various ways in order to obtain live vaccines. Such live vaccines also form part of the invention.

**Diagnostic, Prognostic, Serotypine and Mutation Assays**

This invention is also related to the use of BASB129 polynucleotides and polypeptides for use as diagnostic reagents. Detection of BASB 129 polynucleotides and/or polypeptides in a eukaryote, particularly a mammal, and especially a human, will provide a diagnostic method for diagnosis of disease, staging of disease or response of an infectious organism to drugs. Eukaryotes, particularly mammals, and especially humans, particularly those infected or suspected to be infected with an organism comprising the BASB 129 gene or protein, may be detected at the nucleic acid or amino acid level by a variety of well known techniques as well as by methods provided herein.

Polypeptides and polynucleotides for prognosis, diagnosis or other analysis may be obtained from a putatively infected and/or infected individual's bodily materials. Polynucleotides from any of these sources, particularly DNA or RNA, may be used directly for detection or may be amplified enzymatically by using PCR or any other amplification technique prior to analysis. RNA, particularly mRNA, cDNA and genomic DNA may also be used in the same ways. Using amplification, characterization of the species and strain of infectious or resident organism present in an individual, may be made by an analysis of the genotype of a selected polynucleotide of the organism. Deletions and insertions can be detected by a change in size of the amplified product in comparison to a genotype of a reference sequence selected from a related organism, preferably a different species of the same genus or a different strain of the same species. Point mutations can be identified by hybridizing amplified DNA to labeled BASB129 polynucleotide sequences. Perfectly or significantly matched sequences can be distinguished from imperfectly or more significantly mismatched duplexes by DNase or RNase digestion, for DNA or RNA respectively, or by detecting differences in melting temperatures or renaturation kinetics. Polynucleotide sequence differences may also be detected by alterations in the electrophoretic mobility of polynucleotide fragments in gels as compared to a reference sequence. This may be carried out with or without denaturing agents. Polynucleotide differences may also be detected by direct DNA or RNA sequencing. See, for example, Myers et al., Science, 230: 1242 (1985). Sequence changes at specific locations also may be revealed by nuclease protection assays, such as RNase, V 1 and S1 protection assay or a chemical cleavage method. See, for example, Cotton et al., Proc. Natl. Acad Sci., USA, 85: 4397-4401 (1985).

An array of oligonucleotides probes comprising BASB129 nucleotide sequence or fragments thereof can be constructed to conduct efficient screening of, for example, genetic mutations, serotype, taxonomic classification or identification. Array technology methods are well known and have general applicability and can be used to address a variety of questions in molecular genetics including gene expression, genetic linkage, and genetic variability (see, for example, Chee et al., Science, 274: 610 (1996)).

There is described a diagnostic kit which comprises:

(a) a polynucleotide of the present invention, preferably the nucleotide sequence of SEQ ID NO: 1, or a fragment thereof ;
(b) a nucleotide sequence complementary to that of (a);
(c) a polypeptide of the present invention, preferably the polypeptide of SEQ ID NO:2 or a fragment thereof; or
(d) an antibody to a polypeptide of the present invention, preferably to the polypeptide of SEQ ID NO:2.

It will be appreciated that in any such kit, (a), (b), (c) or (d) may comprise a substantial component. Such a kit will be of use in diagnosing a disease or susceptibility to a Disease, among others.

This invention also relates to the use of the disclosed polynucleotides as diagnostic reagents. Detection of a mutated form of a disclosed polynucleotide, preferably SEQ ID NO:1, which is associated with a disease or pathogenicity will provide a diagnostic tool that can add to, or define, a diagnosis of a disease, a prognosis of a course of disease, a determination of a stage of disease, or a susceptibility to a disease, which results from under-expression, over-expression or altered expression of the polynucleotide. Organisms, particularly infectious organisms, carrying mutations in such polynucleotide may be detected at the polynucleotide level by a variety of techniques, such as those described elsewhere herein.

Cells from an organism carrying mutations or polymorphisms (allelic variations) in a polynucleotide and/or polypeptide described herein may also be detected at the polynucleotide or polypeptide level by a variety of techniques, to allow for serotyping, for example. For example, RT-PCR can be used to detect mutations in the RNA. It is particularly preferred to use RT-PCR in conjunction with automated detection systems, such as, for example, GeneScan. RNA, cDNA or genomic DNA may also be used for the same purpose, PCR. As an example, PCR primers complementary to a polynucleotide encoding BASB129 polypeptide can be used to identify and analyze mutations.

There is also described primers with 1, 2, 3 or 4 nucleotides removed from the 5' and/or the 3' end. These primers may be used for, among other things, amplifying BASB129 DNA and/or RNA isolated from a sample derived from an individual, such as a bodily material. The primers may be used to amplify a polynucleotide isolated from an infected individual, such that the polynucleotide may then be subject to various techniques for elucidation of the polynucleotide sequence. In this way, mutations in the polynucleotide sequence may be detected and used to diagnose and/or prognose the infection or its stage or course, or to serotype and/or classify the infectious agent.

The invention further provides a process for diagnosing infections caused by *Moraxella catarrhalis,* comprising determining from a sample derived from an individual, such as a bodily material, an increased level of expression of polynucleotide having a sequence of SEQ ID NO: 1. Increased or decreased expression of a BASB 129 polynucleotide can be measured using any on of the methods well known in the art for the quantitation of polynucleotides, such as, for example, amplification, PCR, RT-PCR, RNase protection, Northern blotting, spectrometry and other hybridization methods.

In addition, a diagnostic assay for detecting over-expression of BASB 129 polypeptide compared to normal control tissue samples may be used to detect the presence of an infection, for example. Assay techniques that can be used to determine levels of a BASB129 polypeptide, in a sample derived from a host, such as a bodily material, are well-known to those of skill in the art. Such assay methods include radioimmunoassays, competitive-binding assays, Western Blot analysis, antibody sandwich assays, antibody detection and ELISA assays.

The disclosed polynucleotides may be used as components of polynucleotide arrays, preferably high density arrays or grids. These high density arrays are particularly useful for diagnostic and prognostic purposes. For example, a set of spots each comprising a different gene, and further comprising a polynucleotide or polynucleotides of the invention, may be used for probing, such as using hybridization or nucleic acid amplification, using a probes obtained or derived from a bodily sample, to determine the presence of a particular polynucleotide sequence or related sequence in an individual. Such a presence may indicate the presence of a pathogen, particularly *Moraxella catarrhalis,* and may be useful in diagnosing and/or prognosing disease or a course of disease. A grid comprising a number of variants of the polynucleotide sequence of SEQ ID NO:1 are preferred. Also preferred is a comprising a number of variants of a polynucleotide sequence encoding the polypeptide sequence of SEQ ID NO:2.

## Antibodies

The disclosed polypeptides and polynucleotides or variants thereof, or cells expressing the same can be used as immunogens to produce antibodies immunospecific for such polypeptides or polynucleotides respectively. The term "immunospecific" means that the antibodies have substantially greater affinity for the polypeptides of the invention than their affinity for other related polypeptides in the prior art.

In certain preferred embodiments of the invention there are provided antibodies against BASB 129 polypeptide fragments.

Antibodies generated against the disclosed polypeptides or polynucleotides can be obtained by administering the polypeptides and/or polynucleotides, or epitope-bearing fragments of either or both, analogues of either or both, or cells expressing either or both, to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., Nature 256: 495-497 (1975); Kozbor et al., Immunology Today 4: 72 (1983); Cole et al., pg. 77-96 in MONOCLONAL ANTIBODIES AND CANCER THERAPY, Alan R. Liss, Inc. (1985).

Techniques for the production of single chain antibodies (U.S. Patent No. 4,946,778) can be adapted to produce single chain antibodies to polypeptides or polynucleotides of this invention. Also, transgenic mice, or other organisms or animals, such as other mammals, may be used to express humanized antibodies immunospecific to the disclosed polypeptides or polynucleotides.

Alternatively, phage display technology may be utilized to select antibody genes with binding activities towards a disclosed polypeptide either from repertoires of PCR amplified v-genes of lymphocytes from humans screened for possessing anti-BASB 129 or from naive libraries (McCafferty, et al., (1990), Nature 348, 552-554; Marks, et al., (1992) Biotechnology 10, 779-783). The affinity of these antibodies can also be improved by, for example, chain shuffling (Clackson et al., (1991) Nature 352: 628).

The above-described antibodies may be employed to isolate or to identify clones expressing the disclosed polypeptides or polynucleotides to purify the polypeptides or polynucleotides by, for example, affinity chromatography.

Thus, among others, antibodies against BASB 129-polypeptide or BASB 129-polynucleotide may be employed to treat infections, particularly bacterial infections.

Polypeptide variants include antigenically, epitopically or immunologically equivalent variants.

Preferably, the antibody or variant thereof is modified to make it less immunogenic in the individual. For example, if the individual is human the antibody may most preferably be "humanized," where the complimentarity determining region or regions of the hybridoma-derived antibody has been transplanted into a human monoclonal antibody, for example as described in Jones et al. (1986), Nature 321, 522-525 or Tempest et al., (1991) Biotechnology 9, 266-273.

## Antagonists and Agonists - Assays and Molecules

The disclosed polypeptides and polynucleotides may also be used to assess the binding of small molecule substrates and ligands in, for example, cells, cell-free preparations, chemical libraries, and natural product mixtures. These substrates and ligands may be natural substrates and ligands or may be structural or functional mimetics. See, e.g., Coligan et al., Current Protocols in Immunology 1(2): Chapter 5 (1991).

The screening methods may simply measure the binding of a candidate compound to the polypeptide or polynucleotide, or to cells or membranes bearing the polypeptide or polynucleotide, or a fusion protein of the polypeptide by means of a label directly or indirectly associated with the candidate compound. Alternatively, the screening method may involve competition with a labeled competitor. Further, these screening methods may test whether the candidate compound results in a signal generated by activation or inhibition of the polypeptide or polynucleotide, using detection systems appropriate to the cells comprising the polypeptide or polynucleotide. Inhibitors of activation are generally assayed in the presence of a known agonist and the effect on activation by the agonist by the presence of the candidate compound is observed. Constitutively active polypeptide and/or constitutively expressed polypeptides and polynucleotides may be employed in screening methods for inverse agonists or inhibitors, in the absence of an agonist or inhibitor, by testing whether the candidate compound results in inhibition of activation of the polypeptide or polynucleotide, as the case may be. Further, the screening methods may simply comprise the steps of mixing a candidate compound with a solution containing a disclosed polypeptide or polynucleotide, to form a mixture, measuring BASB 129 polypeptide and/or polynucleotide activity in the mixture, and comparing the BASB 129 polypeptide and/or polynucleotide activity of the mixture to a standard. Fusion proteins, such as those made from Fc portion and BASB129 polypeptide, as hereinbefore described, can also be used for high-throughput screening assays to identify antagonists of the disclosed polypeptide, as well as of phylogenetically and and/or functionally related polypeptides (see D. Bennett et al., J Mol Recognition, 8: 52-58 (1995); and K. Johanson et al., J Biol Chem, 270(16):9459-9471 (1995)).

The polynucleotides, polypeptides and antibodies that bind to and/or interact with a polypeptide herein described may also be used to configure screening methods for detecting the effect of added compounds on the production of mRNA and/or polypeptide in cells. For example, an ELISA assay may be constructed for measuring secreted or cell associated levels of polypeptide using monoclonal and polyclonal antibodies by standard methods known in the art. This can be used to discover agents which may inhibit or enhance the production of polypeptide (also called antagonist or agonist, respectively) from suitably manipulated cells or tissues.

There is also described a method of screening compounds to identify those which enhance (agonist) or block (antagonist) the action of BASB 129 polypeptides or polynucleotides, particularly those compounds that are bacteriostatic and/or bactericidal. The method of screening may involve high-throughput techniques. For example, to screen for agonists or antagonists, a synthetic reaction mix, a cellular compartment, such as a membrane, cell envelope or cell wall, or a preparation of any thereof, comprising BASB 129 polypeptide and a labeled substrate or ligand of such polypeptide is incubated in the absence or the presence of a candidate molecule that may be a BASB 129 agonist or antagonist. The ability of the candidate molecule to agonize or antagonize the BASB129 polypeptide is reflected in decreased binding of the labeled ligand or decreased production of product from such substrate. Molecules that bind gratuitously, *i.e.*, without inducing the effects of BASB129 polypeptide are most likely to be good antagonists. Molecules that bind well and, as the case may be, increase the rate of product production from substrate, increase signal transduction, or increase chemical channel activity are agonists. Detection of the rate or level of, as the case may be, production of product from substrate, signal transduction, or chemical channel activity may be enhanced by using a reporter system. Reporter systems that may be useful in this regard include but are not limited to colorimetric, labeled substrate converted into product, a reporter gene that is responsive to changes in BASB 129 polynucleotide or polypeptide activity, and binding assays known in the art.

Another example of an assay for BASB 129 agonists is a competitive assay that combines BASB129 and a potential agonist with BASB129-binding molecules, recombinant BASB 129 binding molecules, natural substrates or ligands, or substrate or ligand mimetics, under appropriate conditions for a competitive inhibition assay. BASB 129 can be labeled, such as by radioactivity or a colorimetric compound, such that the number of BASB 129 molecules bound to a binding molecule or converted to product can be determined accurately to assess the effectiveness of the potential antagonist.

Potential antagonists include, among others, small organic molecules, peptides, polypeptides and antibodies that bind to a polynucleotide and/or polypeptide described herein and thereby inhibit or extinguish its activity or expression. Potential antagonists also may be small organic molecules, a peptide, a polypeptide such as a closely related protein or antibody that binds the same sites on a binding molecule, such as a binding molecule, without inducing BASB 129-induced activities, thereby preventing the action or expression of BASB 129 polypeptides and/or polynucleotides by excluding BASB 129 polypeptides and/or polynucleotides from binding.

Potential antagonists include a small molecule that binds to and occupies the binding site of the polypeptide thereby preventing binding to cellular binding molecules, such that normal biological activity is prevented. Examples of small molecules include but are not limited to small organic molecules, peptides or peptide-like molecules. Other potential antagonists include antisense molecules (see Okano, J. Neurochem. 56: 560 (1991); OLIGODEOXYNUCLEOTIDES AS ANTISENSE INHIBITORS OF GENE EXPRESSION, CRC Press, Boca Raton, FL (1988), for a description of these molecules). Antagonists include compounds related to and variants of BASB 129.

Therei s also described genetically engineered soluble fusion proteins comprising a polypeptide diclosed herein, or a fragment thereof, and various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. The Fc part can be removed simply by incorporation of a cleavage sequence which can be cleaved with blood clotting factor Xa.

There is also described processes for the preparation of these fusion proteins by genetic engineering, and to the use thereof for drug screening, diagnosis and therapy. There is also described polynucleotides encoding such fusion proteins. Examples of fusion protein technology can be found in International Patent Application Nos. WO94/29458 and WO94/22914.

Each of the polynucleotide sequences disclosed herein may be used in the discovery and development of antibacterial compounds. The encoded protein, upon expression, can be used as a target for the screening of antibacterial drugs. Additionally, the polynucleotide sequences encoding the amino terminal regions of the encoded protein or Shine-Delgarno or other translation facilitating sequences of the respective mRNA can be used to construct antisense sequences to control the expression of the coding sequence of interest.

The invention also provides the use of the polypeptide, polynucleotide, agonist or antagonist of the invention to interfere with the initial physical interaction between a pathogen or pathogens and a eukaryotic, preferably mammalian, host responsible for sequelae of infection. In particular, the molecules of the invention may be used: in the prevention of adhesion of bacteria, in particular gram positive and/or gram negative bacteria, to eukaryotic, preferably mammalian, extracellular matrix proteins on in-dwelling devices or to extracellular matrix proteins in wounds; to block bacterial adhesion between eukaryotic, preferably mammalian, extracellular matrix proteins and bacterial BASB129 proteins that mediate tissue damage and/or; to block the normal progression of pathogenesis in infections initiated other than by the implantation of in-dwelling devices or by other surgical techniques.

There is described BASB 129 agonists and antagonists, including bacteristatic or bactericidal agonists and antagonists.

The disclosed antagonists and agonists may be employed, for instance, to prevent, inhibit and/or treat diseases.

There is also described mimotopes of the disclosed polypeptide. A mimotope is a peptide sequence, sufficiently similar to the native peptide (sequentially or structurally), which is capable of being recognised by antibodies which recognise the native peptide; or is capable of raising antibodies which recognise the native peptide when coupled to a suitable carrier.

Peptide mimotopes may be designed for a particular purpose by addition, deletion or substitution of elected amino acids. Thus, the peptides may be modified for the purposes of ease of conjugation to a protein carrier. For example, it may be desirable for some chemical conjugation methods to include a terminal cysteine. In addition it may be desirable for peptides conjugated to a protein carrier to include a hydrophobic terminus distal from the conjugated terminus of the peptide, such that the free unconjugated end of the peptide remains associated with the surface of the carrier protein. Thereby presenting the peptide in a conformation which most closely resembles that of the peptide as found in the context of the whole native molecule. For example, the peptides may be altered to have an N-terminal cysteine and a C-terminal hydrophobic amidated tail. Alternatively, the addition or substitution of a D-stereoisomer form of one or more of the amino acids may be performed to create a beneficial derivative, for example to enhance stability of the peptide.

Alternatively, peptide mimotopes may be identified using antibodies which are capable themselves of binding to the disclosed polypeptides using techniques such as phage display technology (EP 0 552 267 B1). This technique, generates a large number of peptide sequences which mimic the structure of the native peptides and are, therefore, capable of binding to anti-native peptide antibodies, but may not necessarily themselves share significant sequence homology to the native polypeptide.

## Vaccines

Another aspect of the invention relates to a method for inducing an immunological response in an individual, particularly a mammal, preferably humans, which comprises inoculating the individual with BASB129 polynucleotide and/or polypeptide, or a fragment or variant thereof, adequate to produce antibody and/ or T cell immune response to protect said individual from infection, particularly bacterial infection and most particularly *Moraxella catarrhalis* infection. Also provided are methods whereby such immunological response slows bacterial replication. Yet another aspect of the invention relates to a method of inducing immunological response in an individual which comprises delivering to such individual a nucleic acid vector, sequence or ribozyme to direct expression of BASB129 polynucleotide and/or polypeptide, or a fragment or a variant thereof, for expressing BASB 129 polynucleotide and/or polypeptide, or a fragment or a variant thereof *in vivo* in order to induce an immunological response, such as, to produce antibody and/ or T cell immune response, including, for example, cytokine-producing T cells or cytotoxic T cells, to protect said individual, preferably a human, from disease, whether that disease is already established within the individual or not. One example of administering the gene is by accelerating it into the desired cells as a coating on particles or otherwise. Such nucleic acid vector may comprise DNA, RNA, a ribozyme, a modified nucleic acid, a DNA/RNA hybrid, a DNA-protein complex or an RNA-protein complex.

A further aspect of the invention relates to an immunological composition that when introduced into an individual, preferably a human, capable of having induced within it an immunological response, induces an immunological response in such individual to a BASB129 polynucleotide and/or polypeptide encoded therefrom, wherein the composition comprises a recombinant BASB129 polynucleotide and/or polypeptide encoded therefrom and/or comprises DNA and/or RNA which encodes and expresses an antigen of said BASB129 polynucleotide, polypeptide encoded therefrom, or other polypeptide of the invention. The immunological response may be used therapeutically or prophylactically and may take the form of antibody immunity and/or cellular immunity, such as cellular immunity arising from CTL or CD4+ T cells.

A BASB129 polypeptide or a fragment thereof may be fused with co-protein or chemical moiety which may or may not by itself produce antibodies, but which is capable of stabilizing the first protein and producing a fused or modified protein which will have antigenic and/or immunogenic properties, and preferably protective properties. Thus fused recombinant protein, preferably further comprises an antigenic co-protein, such as lipoprotein D from *Haemophilus influenzae,* Glutathione-S-transferase (GST) or beta-galactosidase, or any other relatively large co-protein which solubilizes the protein and facilitates production and purification thereof. Moreover, the co-protein may act as an adjuvant in the sense of providing a generalized stimulation of the immune system of the organism receiving the protein. The co-protein may be attached to either the amino- or carboxy-terminus of the first protein.

In a vaccine composition according to the invention, a BASB 129 polypeptide and/or polynucleotide, or a fragment, or a mimotope, or a variant thereof may be present in a vector, such as the live recombinant vectors described above for example live bacterial vectors.

Also suitable are non-live vectors for the BASB129 polypeptide, for example bacterial outer-membrane vesicles or "blebs". OM blebs are derived from the outer membrane of the two-layer membrane of Gram-negative bacteria and have been documented in many Gram-negative bacteria (Zhou, L et al. 1998. FEMS Microbiol. Lett. 163:223-228) including C. *trachomatis* and C. *psittaci.* A non-exhaustive list of bacterial pathogens reported to produce blebs also includes: *Bordetella pertussis, Borrelia burgdorferi, Brucella melitensis, Brucella ovis, Esherichia coli, Haemophilus influenza, Legionella pneumophila, Moraxella catarrhalis, Neisseria gonorrhoeae, Neisseria meningitidis, Pseudomonas aeruginosa and Yersinia enterocolitica.*

Blebs have the advantage of providing outer-membrane proteins in their native conformation and are thus particularly useful for vaccines. Blebs can also be improved for vaccine use by engineering the bacterium so as to modify the expression of one or more molecules at the outer membrane. Thus for example the expression of a desired immunogenic protein at the outer membrane, such as the BASB 129 polypeptide, can be introduced or upregulated (e.g. by altering the promoter). Instead or in addition, the expression of outer-membrane molecules which are either not relevant (e.g. unprotective antigens or immunodominant but variable proteins) or detrimental (e.g. toxic molecules such as LPS, or potential inducers of an autoimmune response) can be downregulated. These approaches are discussed in more detail below.

The non-coding flanking regions of the BASB129 gene contain regulatory elements important in the expression of the gene. This regulation takes place both at the transcriptional and translational level. The sequence of these regions, either upstream or downstream of the open reading frame of the gene, can be obtained by DNA sequencing. This sequence information allows the determination of potential regulatory motifs such as the different promoter elements, terminator sequences, inducible sequence elements, repressors, elements responsible for phase variation, the shine-dalgarno sequence, regions with potential secondary structure involved in regulation, as well as other types of regulatory motifs or sequences. This sequence is a further aspect of the invention.

This sequence information allows the modulation of the natural expression of the BASB129 gene. The upregulation of the gene expression may be accomplished by altering the promoter, the shine-dalgarno sequence, potential repressor or operator elements, or any other elements involved. Likewise, downregulation of expression can be achieved by similar types of modification. Alternatively, by changing phase variation sequences, the expression of the gene can be put under phase variation control, or it may be uncoupled from this regulation. In another approach, the expression of the gene can be put under the control of one or more inducible elements allowing regulated expression. Examples of such regulation include, but are not limited to, induction by temperature shift, addition of inductor substrates like selected carbohydrates or their derivatives, trace elements, vitamins, co-factors, metal ions, etc.

Such modifications as described above can be introduced by several different means. The modification of sequences involved in gene expression can be carried out *in vivo* by random mutagenesis followed by selection for the desired phenotype. Another approach consists in isolating the region of interest and modifying it by random mutagenesis, or site-directed replacement, insertion or deletion mutagenesis. The modified region can then be reintroduced into the bacterial genome by homologous recombination, and the effect on gene expression can be assessed. In another approach, the sequence knowledge of the region of interest can be used to replace or delete all or part of the natural regulatory sequences. In this case, the regulatory region targeted is isolated and modified so as to contain the regulatory elements from another gene, a combination of regulatory elements from different genes, a synthetic regulatory region, or any other regulatory region, or to delete selected parts of the wild-type regulatory sequences. These modified sequences can then be reintroduced into the bacterium via homologous recombination into the genome. A non-exhaustive list of preferred promoters that could be used for up-regulation of gene expression includes the promoters porA, porB, 1bpB, tbpB, p110, Ist, hpuAB from *N. meningitidis* or *N. gonorroheae;* ompCD, copB, IbpB, ompE, UspA1; UspA2; TbpB from *M Catarrhalis;* p1, p2, p4, p5, p6, IpD, tbpB, D15, Hia, Hmw1, Hmw2 from H. *influenzae.*

In one example, the expression of the gene can be modulated by exchanging its promoter with a stronger promoter (through isolating the upstream sequence of the gene, in vitro modification of this sequence, and reintroduction into the genome by homologous recombination). Upregulated expression can be obtained in both the bacterium as well as in the outer membrane vesicles shed (or made) from the bacterium.

In other examples, the described approaches can be used to generate recombinant bacterial strains with improved characteristics for vaccine applications. These can be, but are not limited to, attenuated strains, strains with increased expression of selected antigens, strains with knock-outs (or decreased expression) of genes interfering with the immune response, strains with modulated expression of immunodominant proteins, strains with modulated shedding of outer-membrane vesicles.

Thus, also provided by the invention is a modified upstream region of the BASB 129 gene, which modified upstream region contains a heterologous regulatory element which alters the expression level of the BASB129 protein located at the outer membrane. The upstream region according to this aspect of the invention includes the sequence upstream of the BASB 129 gene. The upstream region starts immediately upstream of the BASB 129 gene and continues usually to a position no more than about 1000 bp upstream of the gene from the ATG start codon. In the case of a gene located in a polycistronic sequence (operon) the upstream region can start immediately preceding the gene of interest, or preceding the first gene in the operon. Preferably, a modified upstream region according to this aspect of the invention contains a heterologous promotor at a position between 500 and 700 bp upstream of the ATG.

Thus, the invention provides a BASB 129 polypeptide, in a modified bacterial bleb. The invention further provides modified host cells capable of producing the non-live membrane-based bleb vectors. The invention further provides nucleic acid vectors comprising the BASB129 gene having a modified upstream region containing a heterologous regulatory element.

Further provided by the invention are processes to prepare the host cells and bacterial blebs according to the invention.

Also provided by this invention are compositions, particularly vaccine compositions, and methods comprising the polypeptides and/or polynucleotides of the invention and immunostimulatory DNA sequences, such as those described in Sato, Y. *et al.* Science 273: 352 (1996).

Also, provided by this invention are methods using the described polynucleotide or particular fragments thereof, which have been shown to encode non-variable regions of bacterial cell surface proteins, in polynucleotide constructs used in such genetic immunization experiments in animal models of infection with *Moraxella catarrhalis.* Such experiments will be particularly useful for identifying protein epitopes able to provoke a prophylactic or therapeutic immune response. It is believed that this approach will allow for the subsequent preparation of monoclonal antibodies of particular value, derived from the requisite organ of the animal successfully resisting or clearing infection, for the development of prophylactic agents or therapeutic treatments of bacterial infection, particularly *Moraxella catarrhalis* infection, in mammals, particularly humans.

The invention also includes a vaccine formulation which comprises an here described immunogenic recombinant polypeptide and/or polynucleotide together with a suitable carrier, such as a pharmaceutically acceptable carrier. Since the polypeptides and polynucleotides may be broken down in the stomach, each is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteriostatic compounds and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use.

The vaccine formulation of the invention may also include adjuvant systems for enhancing the immunogenisity of the formulation. Preferably the adjuvant system raises preferentially a TH I type of response.

An immune response may be broadly distinguished into two extreme catagories, being a humoral or cell mediated immune responses (traditionally characterised by antibody and cellular effector mechanisms of protection respectively). These categories of response have been termed TH1-type responses (cell-mediated response), and TH2-type immune responses (humoral response).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgGI type antibodies. TH2-type immune responses are characterised by the generation of a broad range of immunoglobulin isotypes including in mice IgGI, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman *(*Mosmann, T.R. and Coffman, R.L. (1989) TH1 and TH2 cells: different patterns of lymphokine secretion lead to different functional properties. Annual Review of Immunology, 7, p145-173*).* Traditionally, TH1-type responses are associated with the production of the INF-γ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH1:TH2 balance of the immune response after a vaccination or infection includes direct measurement of the production of TH 1 or TH2 cytokines by T lymphocytes *in vitro* after restimulation with antigen, and/or the measurement of the IgG1:IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

Adjuvants which are capable of preferential stimulation of the TH1 cell response are described in International Patent Application No. WO 94/00153 and WO 95/17209.

3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem, Montana. A preferred form of 3 De-O-acylated monophosphoryl lipid A is disclosed in European Patent 0 689 454 B1 (SmithKline Beecham Biologicals SA).

Preferably, the particles of 3D-MPL are small enough to be sterile filtered through a 0.22micron membrane (European Patent number 0 689 454). 3D-MPL will be present in the range of 10μg - 100 μg preferably 25-50 μg per dose wherein the antigen will typically be present in a range 2-50 μg per dose.

Another preferred adjuvant comprises QS21, an Hplc purified non-toxic fraction derived from the bark of Quillaja Saponaria Molina. Optionally this may be admixed with 3 De-O-acylated monophosphoryl lipid A (3D-MPL), optionally together with an carrier.

The method of production of QS21 is disclosed in US patent No. 5,057,540.

Non-reactogenic adjuvant formulations containing QS21 have been described previously (WO 96/33739). Such formulations comprising QS21 and cholesterol have been shown to be successful TH1 stimulating adjuvants when formulated together with an antigen.

Further adjuvants which are preferential stimulators of TH1 cell response include immunomodulatory oligonucleotides, for example unmethylated CpG sequences as disclosed in WO 96/02555.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1:1. The preferred range for optimal synergy is 2.5 : 1 to 1 : 1 3D-MPL: QS21.

Preferably a carrier is also present in the vaccine composition according to the invention. The carrier may be an oil in water emulsion, or an aluminium salt, such as aluminium phosphate or aluminium hydroxide.

A preferred oil-in-water emulsion comprises a metabolisible oil, such as squalene, alpha tocopherol and Tween 80. In a particularly preferred aspect the antigens in the vaccine composition according to the invention are combined with QS21 and 3D-MPL in such an emulsion. Additionally the oil in water emulsion may contain span 85 and/or lecithin and/or tricaprylin.

Typically for human administration QS21 and 3D-MPL will be present in a vaccine in the range of 1μg - 200μg, such as 10-100μg, preferably 10μg - 50μg per dose. Typically the oil in water will comprise from 2 to 10% squalene, from 2 to 10% alpha tocopherol and from 0.3 to 3% tween 80. Preferably the ratio of squalene: alpha tocopherol is equal to or less than 1 as this provides a more stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The present invention also provides a polyvalent vaccine composition comprising a vaccine formulation of the invention in combination with other antigens, in particular antigens useful for treating cancers, autoimmune diseases and related conditions. Such a polyvalent vaccine composition may include a TH-1 inducing adjuvant as hereinbefore described.

While the invention has been described with reference to certain BASB129 polypeptides and polynucleotides, it is to be understood that this covers fragments of the naturally occurring polypeptides and polynucleotides, and similar polypeptides and polynucleotides with additions, deletions or substitutions which do not substantially affect the immunogenic properties of the recombinant polypeptides or polynucleotides.

## Compositions, kits and administration

In a further aspect of the invention there are provided compositions comprising a BASB 129 polynucleotide and/or a BASB 129 polypeptide for administration to a cell or to a multicellular organism.

The invention also relates to compositions comprising a polynucleotide and/or a polypeptides discussed herein or their agonists or antagonists. The polypeptides and polynucleotides herein described may be employed in combination with a non-sterile or sterile carrier or carriers for use with cells, tissues or organisms, such as a pharmaceutical carrier suitable for administration to an individual. Such compositions comprise, for instance, a media additive or a therapeutically effective amount of a disclosed polypeptide and/or polynucleotide and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the invention.

The disclosed polypeptides, polynucleotides and other disclosed compounds may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

In a further aspect, the present invention provides for pharmaceutical compositions comprising a therapeutically effective amount of a polypeptide and/or polynucleotide, such as the soluble form of a polypeptide and/or polynucleotide herein described, agonist or antagonist peptide or small molecule compound, in combination with a pharmaceutically acceptable carrier or excipient. Such carriers include, but are not limited to, saline, buffered saline, dextrose, water, glycerol, ethanol, and combinations thereof. There is also described pharmaceutical packs and kits comprising one or more containers filled with one or more of the ingredients of the aforementioned compositions of the invention. Polypeptides, polynucleotides and other compounds disclosed herein may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of salves, pastes, gels, solutions, powders and the like.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

The dosage range required depends on the choice of peptide, the route of administration, the nature of the formulation, the nature of the subject's condition, and the judgment of the attending practitioner. Suitable dosages, however, are in the range of 0.1-100 $\mu$g/kg of subject.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the invention which would preclude their administration to suitable individuals.

Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

## Sequence Databases, Sequences in a Tangible Medium, and Algorithms

Polynucleotide and polypeptide sequences form a valuable information resource with which to determine their 2- and 3-dimensional structures as well as to identify further sequences of similar homology. These approaches are most easily facilitated by storing the sequence in a computer readable medium and then using the stored data in a known macromolecular structure program or to search a sequence database using well known searching tools, such as the GCG program package.

Also provided by the invention are methods for the analysis of character sequences or strings, particularly genetic sequences or encoded protein sequences. Preferred methods of sequence analysis include, for example, methods of sequence homology analysis, such as identity and similarity analysis, DNA, RNA and protein structure analysis, sequence assembly, cladistic analysis, sequence motif analysis, open reading frame determination, nucleic acid base calling, codon usage analysis, nucleic acid base trimming, and sequencing chromatogram peak analysis.

A computer based method is provided for performing homology identification. This method comprises the steps of: providing a first polynucleotide sequence comprising the sequence of a polynucleotide of the invention in a computer readable medium; and comparing said first polynucleotide sequence to at least one second polynucleotide or polypeptide sequence to identify homology.

A computer based method is also provided for performing homology identification, said method comprising the steps of providing a first polypeptide sequence comprising the sequence of a polypeptide of the invention in a computer readable medium; and comparing said first polypeptide sequence to at least one second polynucleotide or polypeptide sequence to identify homology.

All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

## DEFINITIONS

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in *(*Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Methods to determine identity are designed to give the largest match between the sequences tested. Moreover, methods to determine identity are codified in publicly available computer programs. Computer program methods to determine identity between two sequences include, but are not limited to, the GAP program in the GCG program package (Devereux, J., et al., Nucleic Acids Research 12(1): 387 (1984)), BLASTP, BLASTN (Altschul, S.F. et al., J. Molec. Biol. 215: 403-410 (1990), and FASTA( Pearson and Lipman Proc. Natl. Acad. Sci. USA 85; 2444-2448 (1988). The BLAST family of programs is publicly available from NCBI and other sources *(*BLAST Manual, Altschul, S., et al., NCBI NLM NIH Bethesda, MD 20894; Altschul, S., et al., J. Mol. Biol. 215: 403-410 (1990). The well known Smith Waterman algorithm may also be used to determine identity.

Parameters for polypeptide sequence comparison include the following:

Algorithm: Needleman and Wunsch, J. Mol Biol. 48: 443-453 (1970) Comparison matrix: BLOSSUM62 from Henikoff and Henikoff, Proc. Natl. Acad. Sci. USA. 89:10915-10919 (1992)
Gap Penalty: 8
Gap Length Penalty: 2

A program useful with these parameters is publicly available as the "gap" program from Genetics Computer Group, Madison WI. The aforementioned parameters are the default parameters for peptide comparisons (along with no penalty for end gaps).

Parameters for polynucleotide comparison include the following:

Algorithm: Needleman and Wunsch, J. Mol Biol. 48: 443-453 (1970)
Comparison matrix: matches = +10, mismatch = 0
Gap Penalty: 50
Gap Length Penalty: 3
Available as: The "gap" program from Genetics Computer Group, Madison WI. These are the default parameters for nucleic acid comparisons.

A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to the reference sequence of SEQ ID NO:1, wherein said polynucleotide sequence may be identical to the reference sequence of SEQ ID NO: 1 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in SEQ ID NO:1 by the integer defining the percent identity divided by 100 and then subtracting that product from said total number of nucleotides in SEQ ID NO: 1, or:

$$n_n \leq x_n - (x_n \bullet y),$$

wherein $n_n$ is the number of nucleotide alterations, $x_n$ is the total number of nucleotides in SEQ ID NO: 1, y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $x_n$ and y is rounded down to the nearest integer prior to subtracting it from $x_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.
By way of example, a polynucleotide sequence of the present invention may be identical to the reference sequence of SEQ ID NO: 1, that is it may be 100% identical, or it may include up to a certain integer number of nucleic acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of nucleic acids in SEQ ID NO: I by the integer defining the percent identity divided by 100 and then subtracting that product from said total number of nucleic acids in SEQ ID NO:1, or:

$$n_n \leq x_n - (x_n \bullet y),$$

wherein $\mathbf{n}_n$ is the number of nucleic acid alterations, $\mathbf{x}_n$ is the total number of nucleic acids in SEQ ID NO:1, **y** is, for instance 0.70 for 70%, 0.80 for 80%, 0.85 for 85% etc., • is the symbol for the multiplication operator, and wherein any non-integer product of $\mathbf{x}_n$ and **y** is rounded down to the nearest integer prior to subtracting it from $\mathbf{x}_n$.

(2) Polypeptide embodiments further include an isolated polypeptide comprising a polypeptide having at least a 50,60, 70, 80, 85, 90, 95, 97 or 100% identity to a polypeptide reference sequence of SEQ ID NO:2, wherein said polypeptide sequence may be identical to the reference sequence of SEQ ID NO:2 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, y is 0.50 for 50%, 0.60 for 60%, 0.70 for 70%, 0.80 for 80%, 0.85 for. 85%, 0.90 for 90%, 0.95 for 95%, 0.97 for 97% or 1.00 for 100%, and • is the symbol for the multiplication operator, and wherein any non-integer product of $\mathbf{x_a}$ and y is rounded down to the nearest integer prior to subtracting it from $\mathbf{x_a}$.

By way of example, a polypeptide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, **y** is, for instance 0.70 for 70%, 0.80 for 80%, 0.85 for 85% etc., and • is the symbol for the multiplication operator, and wherein any non-integer product of $\mathbf{x_a}$ and **y** is rounded down to the nearest integer prior to subtracting it from $\mathbf{x_a}$.

"Individual(s)," when used herein with reference to an organism, means a multicellular eukaryote, including, but not limited to a metazoan, a mammal, an ovid, a bovid, a simian, a primate, and a human.

"Isolated" means altered "by the hand of man" from its natural state, *i.e.,* if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide(s)" generally refers to any polyribonucleotide or polydeoxyribonucleotide, which may be unmodified RNA or DNA or modified RNA or DNA including single and double-stranded regions.

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Disease(s)" means any disease caused by or related to infection by a bacteria, including, for example, otitis media in infants and children, pneumonia in elderlies, sinusitis, nosocomial infections and invasive diseases, chronic otitis media with hearing loss, fluid accumulation in the middle ear, auditive nerve damage, delayed speech learning, infection of the upper respiratory tract and inflammation of the middle ear.

**EXAMPLES:**

The examples below are carried out using standard techniques, which are well known and routine to those of skill in the art, except where otherwise described in detail. The examples are illustrative, but do not limit the invention.

**Example 1: DNA sequencing of the BASB 129 gene from *Moraxella catarrhalis***

**strain ATCC 43617.**

The BASB 129 gene of SEQ ID NO: 1 is from *Moraxella catarrhalis* strain ATCC 43617. The translation of the BASB 129 polynucleotide sequence is showed in SEQ ID NO:2.

**Example 2: Construction of Plasmid to Express Recombinant BASB129**

A: Cloning of BASB129.

The *Eco*RI and *Sal*I restriction sites engineered into reverse amplification primers, permit directional cloning of a PCR product into the *E.coli* expression plasmid pTLZ2 such that a mature BASB 129 protein can be expressed as a fusion protein containing a (His)6 affinity chromatography tag at the C-terminus. The BASB 129 PCR product is purified from the amplification reaction using silica gel-based spin columns (QiaGen) according to the manufacturers instructions. To produce the required *Eco*RI and *Sal*I termini necessary for cloning, purified PCR product is sequentially digested to completion with *Eco*RI and *Sal*I restriction enzymes as recommended by the manufacturer (Life Technologies). Following the first restriction digestion, the PCR product is purified via spin column as above to remove salts and eluted in sterile water prior to the second enzyme digestion. The digested DNA fragment is again purified using silica gel-based spin columns prior to ligation with the pTLZ2 plasmid.

B: Production of Expression Vector.

To prepare the expression plasmid pTLZ2 for ligation, it is similarly digested to completion with both *Eco*RI and *Sal*I and then treated with calf intestinal phosphatase (CIP, ~0.02 units / pmole of 5' end, Life Technologies) as directed by the manufacturer to prevent self ligation. An approximately 5-fold molar excess of the digested fragment to the prepared vector is used to program the ligation reaction. A standard ~20 μl ligation reaction (~16°C, ~16 hours), using methods well known in the art, is performed using T4 DNA ligase (~2.0 units / reaction, Life Technologies). An aliquot of the ligation (~5 μl) is used to transform electro-competent JM109 cells according to methods well known in the art. Following a ~2-3 hour outgrowth period at 37°C in ~1.0 ml of LB broth, transformed cells are plated on LB agar plates containing ampicillin (100 pg/ml). Antibiotic is included in the selection. Plates are incubated overnight at 37°C for ~16 hours. Individual ApR colonies are picked with sterile toothpicks and used to "patch" inoculate fresh LB ApR plates as well as a ~1.0 ml LB ApR broth culture. Both the patch plates and the broth culture are incubated overnight at 37°C in either a standard incubator (plates) or a shaking water bath. A whole cell-based PCR analysis is employed to verify that transformants contain the BASB129 DNA insert. Here, the ~1.0 ml overnight LB Ap broth culture is transferred to a 1.5 ml polypropylene tube and the cells collected by centrifugation in a Beckmann microcentrifuge (~3 min., room temperature, ~12,000 X g). The cell pellet is suspended in ~200μl of sterile water and a ~10μl aliquot used to program a ~50μl final volume PCR reaction containing both BASB 129 forward and reverse amplification primers. Final concentrations of the PCR reaction components are essentially the same as those specified in example 2 except ~5.0 units of *Taq* polymerase is used. The initial 95°C denaturation step is increased to 3 minutes to ensure thermal disruption of the bacterial cells and liberation of plasmid DNA. An ABI Model 9700 thermal cycler and a 32 cycle, three-step thermal amplification profile, i.e. 95°C, 45sec; 55-58°C, 45sec, 72°C, 1min., are used to amplify the BASB129 fragment from the lysed transformant samples. Following thermal amplification, a ~20μl aliquot of the reaction is analyzed by agarose gel electrophoresis (0.8 % agarose in a Tris-acetate-EDTA (TAE) buffer). DNA fragments are visualized by UV illumination after gel electrophoresis and ethidium bromide staining. A DNA molecular size standard (1 Kb ladder, Life Technologies) is electrophoresed in parallel with the test samples and is used to estimate the size of the PCR products. Transformants that produced the expected size PCR product are identified as strains containing a BASB 129 expression construct. Expression plasmid containing strains are then analyzed for the inducible expression of recombinant BASB129.

C: Expression Analysis of PCR-Positive Transformants.

For each PCR-positive transformant identified above, ~5.0 ml of LB broth containing ampicillin (100 μg/ml) is inoculated with cells from the patch plate and grown overnight at 37 °C with shaking (~250 rpm). An aliquot of the overnight seed culture (~1.0 ml) is inoculated into a 125 ml erlenmeyer flask containing ~25 of LB Ap broth and grown at 37 °C with shaking (~250 rpm) until the culture turbidity reaches O.D.600 of ~0.5, i.e. mid-log phase (usually about 1.5 - 2.0 hours). At this time approximately half of the culture (~12.5 ml) is transferred to a second 125 ml flask and expression of recombinant BASB 129 protein induced by the addition of IPTG (1.0 M stock prepared in sterile water, Sigma) to a final concentration of 1.0 mM. Incubation of both the IPTG-induced and non-induced cultures continues for an additional ~4 hours at 37 °C with shaking. Samples (~1.0 ml) of both induced and non-induced cultures are removed after the induction period and the cells collected by centrifugation in a microcentrifuge at room temperature for ~3 minutes. Individual cell pellets are suspended in ~50μl of sterile water, then mixed with an equal volume of 2X Laemelli SDS-PAGE sample buffer containing 2-mercaptoethanol, and placed in boiling water bath for ~3 min to denature protein. Equal volumes (~15μl) of both the crude IPTG-induced and the non-induced cell lysates are loaded onto duplicate 12% Tris/glycine polyacrylamide gel (1 mm thick Mini-gels, Novex). The induced and non-induced lysate samples are electrophoresed together with prestained molecular weight markers (SeeBlue, Novex) under conventional conditions using a standard SDS/Tris/glycine running buffer (BioRad). Following electrophoresis, one gel is stained with commassie brilliant blue R250 (BioRad) and then destained to visualize novel BASB129 IPTG-inducible protein(s). The second gel is electroblotted onto a PVDF membrane (0.45 micron pore size, Novex) for ~2 hrs at 4 °C using a BioRad Mini-Protean II blotting apparatus and Towbin's methanol (20 %) transfer buffer. Blocking of the membrane and antibody incubations are performed according to methods well known in the art. A monoclonal anti-RGS (His)3 antibody, followed by a second rabbit anti-mouse antibody conjugated to HRP (QiaGen), is used to confirm the expression and identity of the BASB 129 recombinant protein. Visualization of the anti-His antibody reactive pattern is achieved using either an ABT insoluble substrate or using Hyperfilm with the Amersham ECL chemiluminescence system.

**Example 3: Production of Recombinant BASB 129**

Bacterial strain

A recombinant expression strain of *E. coli* JM 109 containing a plasmid (pTLZ2) encoding BASB129 from *M. catarrhalis.* is used to produce cell mass for purification of recombinant protein. The expression strain is cultivated on LB agar plates containing 100μg/ml ampicillin ("Ap") to ensure that the pTLZ2 was maintained. For cryopreservation at -80 °C, the strain is propagated in LB broth containing the same concentration of antibiotics then mixed with an equal volume of LB broth containing 30% (w/v) glycerol.

Media

The fermentation medium used for the production of recombinant protein consists of 2X YT broth (Difco) containing 100μg/ml Ap. Antifoam is added to medium for the fermentor at 0.25 ml/L (Antifoam 204, Sigma). To induce expression of the BASB129 recombinant protein, IPTG (Isopropyl B-D-Thiogalactopyranoside) is added to the fermentor (1 mM, final).

Fermentation

A 500-ml erlenmeyer seed flask, containing 50ml working volume, is inoculated with 0.3 ml of rapidly thawed frozen culture, or several colonies from a selective agar plate culture, and incubated for approximately 12 hours at 37 ± 1 °C on a shaking platform at 150rpm (Innova 2100, New Brunswick Scientific). This seed culture is then used to inoculate a 5-L working volume fermentor containing 2X YT broth and both Ap antibiotics. The fermentor (Bioflo 3000, New Brunswick Scientific) is operated at 37 ± 1°C, 0.2 - 0.4 VVM air sparge, 250 rpm in Rushton impellers. The pH is not controlled in either the flask seed culture or the fermentor. During fermentation, the pH range is 6.5 to 7.3 in the fermentor. IPTG (1.0 M stock, prepared in sterile water) is added to the fermentor when the culture reaches mid-log of growth (~0.7 O.D.600 units). Cells are induced for 2 - 4 hours then harvested by centrifugation using either a 28RS Heraeus (Sepatech) or RC5C superspeed centrifuge (Sorvall Instruments). Cell paste is stored at -20 C until processed.

**Example 4: Purification of recombinant BASB 129 from E. coli Extraction-Purification**

Cell paste from IPTG induced culture is resuspended in 60 ml of phosphate buffer pH 7.5 containing 1mM AEBSF and 1mM Aprotinin as protease inhibitors. Cells are lysed in a cell disruptor, centrifuged, washed and re-centrifuged. The pellet is suspended in 100 mM NaH2PO4, 10 mM Tris-HCl buffer pH 8 containing 6M Guanidium Chloride (buffer A) and left for 1 hour at room temperature. Total extract is centrifuged at 27,000g for 20 minutes. Supernatant is incubated for 1 hour at room temperature with Ni-NTA superflow resin equilibrated in buffer A. Resin is washed twice with 100 mM NaH2PO4, 10 mM Tris-HCl buffer pH 6.3, containing 8M Urea (buffer B). Elution is performed successively with buffer B adjusted to pH 5.9 then pH 4.5. Fractions containing BASB121 antigen are neutralized with 25% volume of 0.2M phosphate buffer pH 7.5. Pooled fractions are dialysed successively against 100 mM NaH2PO4 containing 8M Urea, then 4M Urea, then 2M Urea and finally against PBS pH 7.4 containing 0.1 % Triton X-100.
Purified BASB 129 antigen is quantified using Micro BCA assay reagent.

## Example 5: Production of Antisera to Recombinant BASB129

Polyvalent antisera directed against the BASB129 protein are generated by vaccinating rabbits with the purified recombinant BASB129 protein. Animals are bled prior to the first immunization ("pre-bleed") and after the last immunization.
Anti-BASB 129 protein titres are measured by an ELISA using purified recombinant BASB129 protein. The titre is defined as mid-point titers calculated by 4-parameter logistic model using the XL Fit software.
The antisera are also used as the first antibody to identify the protein in a western blot as described in example 7 below. The western-blot is used to demonstrate the presence of anti-BASB 129 antibody in the sera of immunized animals.

## Example 6: Immunological characterization: Surface exposure of BASB129

Anti-BASB 129 protein titres are determined by an ELISA using formalin-killed whole cells of *Moraxella catarrhalis.* The titre is defined as mid-point titers calculated by 4-parameter logistic model using the XL Fit software.
The titre observed with the rabbit or mouse immune sera demonstrates that the BASB129 protein is present at the surface of *M. catarrhalis* cells.

## Example 7. Immunological Characterisation: Western Blot Analysis

Several strains of *M catarrhalis* including ATCC 43617, as well as clinical isolates from various geographic regions, are grown on Muller Hinton agar plates for 24 hours at 36°C. Several colonies are used to inoculate broth. Cultures are grown until the A620 is approximately 0.6 and cells are collected by centrifugation. Cells are then concentrated and solubilized in PAGE sample buffer. The solubilized cells are then resolved on 4-20% polyacrylamide gels and the separated proteins are electrophoretically transferred to PVDF membranes. The PVDF membranes are then pretreated with saturation buffer. All subsequent incubations are carried out using this pretreatment buffer.
PVDF membranes are incubated with preimmune serum or rabbit or mouse immune seru. PVDF membranes are then washed.
PVDF membranes are incubated with biotin-labeled sheep anti-rabbit or mouse Ig. PVDF membranes are then washed 3 times with wash buffer, and incubated with streptavidin-peroxydase. PVDF membranes are then washed 3 times with wash buffer and developed with 4-chloro-1-naphtol.
Detection of a protein corresponding to BASB129 expected molecular weight that is reactive with the antisera is used to demonstrate that this protein is produced by and conserved in all *Moraxella* strains tested.

## Example 8: Immunological characterization: Bactericidal Activity

Complement-mediated cytotoxic activity of anti-BASB 129 antibodies is examined to determine the vaccine potential of BASB 129 protein antiserum that is prepared as described above. The activities of the pre-immune serum and the anti-BASB 129 antiserum in mediating complement killing of *M. catarrhalis* are examined. Strains of *M.catarrhalis* are grown on plates. Several colonies are added to liquid medium. Cultures are grown and collected until the A620 is approximately 0.4. After one wash step, the pellet is suspended and diluted.
Preimmune sera and the anti-BASB 129 sera is deposited into the first well of a 96-wells plate and serial dilutions are deposited in the other wells of the same line. Live diluted *M.catarrahlis* is subsequently added and the mixture is incubated. Complement is added into each well at a working dilution defined beforehand in a toxicity assay.
Each test includes a complement control (wells without serum containing active or inactivated complement source), a positive control (wells containing serum with a know titer of bactericidal antibodies), a culture control (wells without serum and complement) and a serum control (wells without complement).
Bactericidal activity of rabbit or mice antiserum (50% killing of homologous strain) is measured.

## Example 9: Presence of Antibody to BASB129 in Human Convalescent Sera

Western blot analysis of purified recombinant BASB129 is performed as described in Example 7 above, except that a pool of human sera from children infected by *M. catarrhalis* is used as the first antibody preparation. This is used to show that antisera from naturally infected individuals react to the purified recombinant protein.

**Example 10: Efficacy of BASB129 vaccine: enhancement of lung clearance of *M. catarrhalis* in mice.**

This mouse model is based on the analysis of the lung invasion by *M. catarrhalis* following a standard intranasal challenge to vaccinated mice.

Groups of mice are immunized with BASB129 vaccine. After the booster, the mice are challenged by instillation of bacterial suspension into the nostril under anaesthesia.

Mice are killed between 30 minutes and 24 hours after challenge and the lungs are removed aseptically and homogenized individually. The log10 weighted mean number of CFU/lung is determined by counting the colonies grown on agar plates after plating of dilutions of the homogenate. The arithmetic mean of the log10 weighted mean number of CFU/lung and the standard deviations are calculated for each group.

Results are analysed statistically.

In this experiment groups of mice are immunized either with BASB129 or with a killed whole cells (kwc) preparation of *M catarrhalis* or sham immunized.

This is used to show that the kwc preparation and the BASB 129 vaccine induce significant lung clearance as compared to the control group.

**Example 11: Inhibition of *M. catarrhalis* adhesion onto cells by anti-BASB1 29 antiserum.**

This assay measures the capacity of anti BASB 129 sera to inhibit the adhesion of *Moraxella* bacteria to epithelial cells. This activity could prevent colonization of f.i. the nasopharynx by *Moraxella.*

One volume of bacteria is incubated on ice with one volume of pre-immune or anti-BASB 129 immune serum dilution. This mixture is subsequently added in the wells of a 24 well plate containing a confluent cells culture that is washed once with culture medium to remove traces of antibiotic. The plate is centrifuged and incubated.

Each well is then gently washed. After the last wash, sodium glycocholate is added to the wells. After incubation, the cell layer is scraped and homogenised. Dilutions of the homogenate are plated on agar plates and incubated. The number of colonies on each plate is counted and the number of bacteria present in each well calculated.

This is used to show that bacteria incubated with anti-BASB129 antiserum are inhibited in their adherence capacity to the Hep-2 cells.

**Deposited materials**

A deposit containing a *Moraxella catarrhalis* Catlin strain has been deposited with the American Type Culture Collection (herein "ATCC") on June 21, 1997 and assigned deposit number 43617. The deposit was described as *Branhamella catarrhalis* (Frosch and Kolle) and is a freeze-dried, 1.5-2.9 kb insert library constructed from M. catarrhalis isolate obtained from a transtracheal aspirate of a coal miner with chronic bronchitits. The deposit is described in Antimicrob. Agents Chemother. 21: 506-508 (1982).

The *Moraxella catarrhalis* strain deposit is referred to herein as "the deposited strain" or as "the DNA of the deposited strain."

The deposited strain contains a full length BASB129 gene.

A deposit of the vector pMC-D15 consisting of *Moraxella catarrhalis* DNA inserted in pQE30 has been deposited with the American Type Culture Collection (ATCC) on February 12 1999 and assigned deposit number 207105.

The sequence of the polynucleotides contained in the deposited strain / clone, as well as the amino acid sequence of any polypeptide encoded thereby, are controlling in the event of any conflict with any description of sequences herein.

The deposit of the deposited strains have been made under the terms of the Budapest Treaty on the International Recognition of the Deposit of Micro-organisms for Purposes of Patent Procedure. The deposited strains will be irrevocably and without restriction or condition released to the public upon the issuance of a patent. The deposited strains are provided merely as convenience to those of skill in the art and are not an admission that a deposit is required for enablement, such as that required under 35 U.S.C. § 112.

## SEQUENCE INFORMATION

### BASB129, BASB130 and BASB131 Polynucleotide and Polypeptide Sequences

**SEQ ID NO: 1**

***Moraxella catarrhalis* BASB129 polynucleotide sequence from strain ATCC43617**

ATGAAAGCACAATTTACAACCCTAAAAGTCATCGGCGGACTATTTGTAGGTGGCATGATGCTAAATGCACATGCAGCACC
TGAATTATATGGTTACGCAAATCTTGTCGTTTCTGCTAATCATAAAAAAACAACCAACAAGACAACCAATGTCAGTACAT
CAACAACAGACCGCCCCTATCTGTATAGCAGCGGATCACGCATTGGCCTTAGAGGGTCTGAAAAGTTAAATGATGATTAT
GAGGTTTTGTACAAGTTGGAGTACCGTCTAGAAAATGATGGTGATTTACGCAATGAAAAAATCAAGCAGCCAGATGGTAC
TGAAAAGACTGTTGCTAAAACACGCAATTTTGAGGCTCGTGATTCATGGATTGGCGTGAAGCATAAAAAGTATGGTACCA
TCAAGGCGGGTCGTATGTTGTCTTTGGATCCATATGTGCGTTATACTGCCTATTTGGCGTCAGGCGTAGATGGGGTGCGT
ACCAATAATACCATTGCATACGAATCACCAAAAATCAAAGATGTTAGTTTTCAGGCGATGTACATCTTAGATGAAAATAA
AGAGACAGATACCATTGATCGTGATGGTTATTCATTGCTTGTCAAGAAAAACACAGATACATATAATGTTGGTGCAGCTT
ATGCCTATTTTGGTAAAGCAAAAACCTCTTATGGCAAGATTAACTATACTGCGCGTGTGACAGGTAATTATAAAATTAAT
GAGGATTATAAAGTAGGTGGTATCTATCAGCATGTCGGCTATGCCAATGACGACAGTGCCAAAAATAATACAGAACAAGC
TGTTGGCGTGGCTTTGCAGCATTTTAAAGACAAATGGACTCATACCGCACACATGAATCTTGTGAATAATCCTAGTGGGA
AAAAGGGCGATGGGTTTGAGCTGATCGGAGCAATTGACCGTGATATTTCTAAAAATGTCTCTGCAGGCATGGATATCACC
TATGGTAACTTTAACTACGCAACAGAAAAAGAATCTTACATCAACCCAACTATCTATGCGACTGTATATTTTTAA

**SEQ ID NO:2**

***Moraxella catarrhalis* BASB129 polypeptide sequence deduced from the polynucleotide of SEQ ID NO:1**

MKAQFTTLKVIGGLFVGGMMLNAHAAPELYGYANLVVSANHKKTTNKTTNVSTSTTDRPYLYSSGSRIGLRGSEKLNDDYE
VLYKLEYRLENDGDLRNEKIKQPDGTEKTVAKTRNFEARDSWIGVKHKKYGTIKAGRMLSLDPYVRYTAYLASGVDGVRTN
NTIAYESPKIKDVSFQAMYILDENKETDTIDRDGYSLLVKKNTDTYNVGAAYAYFGKAKTSYGKINYTARVTGNYKINEDY
KVGGIYQHVGYANDDSAKNNTEQAVGVALQHFKDKWTHTAHMNLVNNPSGKKGDGFELIGAIDRDISKNVSAGMDITYGNF
NYATEKESYINPTIYATVYF

**SEQ ID NO:3**

***Moraxella catarrhalis* BASB130 polynucleotide sequence from strain ATCC43617**

ATGCGTTTTATACCTTTTGCTGTAACTCTTTTATCTTTTACTGGCTCCTCCATCTTTTCTGTGGCCAAAGCTCAGATTGC
ACCAGACCCAACCAAAGCAAATCTTTCTGCAATAACCGATGATCGTCGTCAGGCGGCTCTTGCACATTTAGCAAGACAAG
ATTTGGCAGCAACAGATGACGACATGGGTGTCGATATCAATCAAACCAATATCAACCAGCCAAATTGGCAAGATGACAAG
AATGGCGATTCATGTTTTTTGGTTGATGAGATTGACTTTGTGATTGAGGGTGGAGAAGATAAACATATTCTAGGTATGAC
GCCGAGCAATTTACGCAGCTTGCTATCGCCCCTGTTGGACCGGACAAAGCCAACATATGCTCTTAATCACTGCATCAACA
ATCACAACCTATCTTTAATTGTAGATATTGCCCATAATGAATTACTAAAGCGTGGTTATTTAACTAGCAGTATTAGCATT
GAAGAGCAGGATTTATCAACTAAAAAACTCACTTTAACAGTCCATGCTGGCAAGGTGAAAAAGGTGATATTGGCCGATGC
TAGCAAAACGCCAACTTATGTCAAAGCCGCCATTCCTCTTAAGTTCAATCAAGCCTTTCGTCTGTCATATTTAGAACAAG
GTCTAGATAATTTAAAACGCATCGATCCAACGGCAACCGTGCAGATTATACCAAGCAGCTCCAGTAATGTCGCCAATGAT
ATTGACATTAACACCACAAACCTTGGTTTTAGTGATTTACTCATTAGGATGGACCGCAGTCAAAGCGCTGTATTTGGCAT
TAATATTGATAACAGTTTATCCAAAGATTATGGCAATTATCTGATTTCTGCTAATGTTCGTGCTAACAACCTCATGCATC
TTAACGATGAATGGAATCTTTCTGCCAACTATCCATTGGCGCGCCTGATTGACGCTGCCCAAAATGATCTTGGGGTTCAG
GTGGGCAAGGATAGGCAGGTTAATTATCACGCTTCCTTGACTATTCCTTATGGATTGTATAAATTTTCTGCCACACACAG
CCACCACCAATACCAGCAATTTCTTGAAGGGCTTCATGCGCCCTTGACTTATCATGGCACCAGCAAGACAAGCTCAATTG

GATTGTCTCGTTTATTGCACCGTGATGGCAATCAAAAGACTGAAGGTTATATAAAGGTTAATCACAAACGAAGCAGTAAT
TATATTGATGATGTTAATCTTGAAGTGCAAAACCGCCGCACAACAGGATATAACATTGGCATCACTCATCAGCATCATCT
TCACCAAGGTGGTTATTTGTATGCCAACTTGGATTATAAGCAAGGAACAGGGGCACTAAAAGCAAAGCCTGCACCTGAAG
AGCATATTTATGATGCTTTTGGGCGACAACTACCCAGTGAGGGTTTTGCAAAAGCACCAATTTGGTCACTATACACCAGT
TTCCAAAAGCCATTTGTGTTAAACAACCCAAATGATACTGAACAAGCGCAAGATAAAGCAACAGCAAATAAACCCATTTA
CACATCCATTCCTCTCACTTATACTGCTAGATTACAAGCCCAATATGCCAAACAACTTCCTGTACCATCTGATTTATTCT
ATTTGGGTGGCAGATATAGCATTAAAGGCATTAAAGAGGGAAATTATTTATCAGGAGAACATGGCTTTAGCCTATCCCAA
GAACTTGCTTGGCAATTGCCATTACAGAATCTTAATCAGCACTTTAGTACAAATGCAAACAGCGCCCAGTTGTATGCAAG
TATTGATCAAGGCTATGCCTATGGAAAAAACACTCTTAATAATCAACGCCATATCTTGGCTGGAGCGGTTGGTATGAGGT
ATTATTTTCAAGGCAGTCAAGATCCAAGAATTCAAGAAACACAAAATGGCTTGACTCATTTCAAAGAATCAAATACTTAT
CTAAATAACACACCAACCACAGCTCATTTGGATATATTCATTGGAAAAGGAATTAAGACGCCTGAATTTATGAAGAAAGA
AACCGTCGTGGGTGTGAGTGCCAGCATTGAGTTTTAA

**SEQ ID NO:4**

***Moraxella catarrhalis*** **BASB130 polypeptide sequence deduced from the polynucleotide of SEQ ID NO:3**

MRFIPFAVTLLSFTGSSIFSVAKAQIAPDPTKANLSAITDDRRQAALAHLARQDLAATDDDMGVDINQTNINQPNWQDDKN
GDSCFLVDEIDFVIEGGEDKHILGMTPSNLRSLLSPLLDRTKPTYALNHCINNHNLSLIVDIAHNELLKRGYLTSSISIEE
QDLSTKKLTLTVHAGKVKKVILADASKTPTYVKAAIPLKFNQAFRLSYLEQGLDNLKRIDPTATVQIIPSSSSNVANDIDI
NTTNLGFSDLLIRMDRSQSAVFGINIDNSLSKDYGNYLISANVRANNLMHLNDEWNLSANYPLARLIDAAQNDLGVQVGKD
RQVNYHASLTIPYGLYKFSATHSHHQYQQFLEGLHAPLTYHGTSKTSSIGLSRLLHRDGNQKTEGYIKVNHKRSSNYIDDV
NLEVQNRRTTGYNIGITHQHHLHQGGYLYANLDYKQGTGALKAKPAPEEHIYDAFGRQLPSEGFAKAPIWSLYTSFQKPFV
LNNPNDTEQAQDKATANKPIYTSIPLTYTARLQAQYAKQLPVPSDLFYLGGRYSIKGIKEGNYLSGEHGFSLSQELAWQLP
LQNLNQHFSTNANSAQLYASIDQGYAYGKNTLNNQRHILAGAVGMRYYFQGSQDPRIQETQNGLTHFKESNTYLNNTPTTA
HLDIFIGKGIKTPEFMKKETVVGVSASIEF

**SEQ ID NO:5**

***Moraxella catarrhalis*** **BASB131 polynucleotide sequence from strain ATCC43617**

ATGAAAATGCCCTATTTTTTGCTTTTGGGATTATTGTTAGTACAGCAGGCTGATGCTGATTATAGCTTGCTTGATGCCCA
AAAACAGCTCATGAGCAGCAGCCCTGCCATCCAAGCAAGTCATGCCTTGTATCAAGCTGATGTCTTACAAGCAGAATCAT
TACAAAAATTACACTATCCTAGGATATCGCTCAATGCACACGCCTTTGTACTGCAACAAAACAGCAGCATACCCTTAGAT
CATATCAAAGAGCAGACCGCCCAGCACATTAACACCCATTTTGACCATCGCTTTGGCAGTGCCCCCGATGGTCTGTTGGA
TGCTTTGCACAACAGCACCCAAACCGCCCTTGACCGCCTGCCTGACCATCAAGATGTCAAGTTACGCCATGATGGCATTA
CCCCAAACATTACCGCCACGATACCCATCTATACAGGTGGCTTAATCAGCAGCACCAAAAATATCGCCAATCTACAAGCA
CAGCGTGGTAAATTTGGATTACAAGAACGCATCTCTTTGGCAAAACTTAATTTGATTCGCCATTATTTTAATGTGCAATT
ACAAAAGCAGCTGACCGACACACAACAAAACATGCTTAGTGCCATGCAGTTACATGTAGATAATGCTTATAAATTAGAAC
AGCAAGGTTTTATCAGTCGTGGACAGCGTATGCAATTTGAAGTGGCACGCAATCAGGTACAAAGACTGTATCAAAGCACC
CAAAATCAGCACCAAAACAGCATTTATGAACTTGCTGTTTTGCTTGGTTTGCCCCACATTGAACCGCTAAGCACACCGCT
GTTTATCAACACCCAGCATCGCCCCAATTGGCAAGCATTACTCAAAGACTCCCAAAATACACCATTAAATCAAAAGCTAA
AAACCGACATTTTGCTTGCCGATGAAAATATCGCCCTAAGACAATCCACCAAAAAACCAAAAATTGCCGCTGTGGCTCGT
TATACCTTAGATGATAAGCCTGATTGGTTTGCAGGTGTGGCGGTTTCTTACAACCTATTCTTGGGCATTGACCGTGATAA
GCAGATTGGGGCAGCACACCTACAAAAACAAGCAGCCCAATTGGGTCATGAACAAGTCAATGAACACATCACAACCACCA
TGCACACCGCTTATGGCGAGATGGTGCTGGCACAAAAAACCCACGCCCTACTACAACAAAATCGTCAAGCCGCCAAAGAA
AATTTGCGTATCCAGACGCTGTCTTTTCAAGAAGGGTTTGGGACGGTGGCAGGTGTGGTTGATGCCCAAAGTGCTTTATC
ACAGATTGACAGCGAAACCGCTCTAAATGCGTATCGCTACCTTTTGGCACTGGCGACGCTGTTGCATTACACAGGGACAA
TTGATGACTTTGATGATTATTTGTCTTTGGATGATGCTCATAAACTGTGA

**SEQ ID NO:6**

***Moraxella catarrhalis*** **BASB131 polypeptide sequence deduced from the polynucleotide of SEQ ID NO:5**

MKMPYFLLLGLLLVQQADADYSLLDAQKQLMSSSPAIQASHALYQADVLQAESLQKLHYPRISLNAHAFVLQQNSSIPLDH
IKEQTAQHINTHFDHRFGSAPDGLLDALHNSTQTALDRLPDHQDVKLRHDGITPNITATIPIYTGGLISSTKNIANLQAQR
GKFGLQERISLAKLNLIRHYFNVQLQKQLTDTQQNMLSAMQLHVDNAYKLEQQGFISRGQRMQFEVARNQVQRLYQSTQNQ
HQNSIYELAVLLGLPHIEPLSTPLFINTQHRPNWQALLKDSQNTPLNQKLKTDILLADENIALRQSTKKPKIAAVARYTLD
DKPDWFAGVAVSYNLFLGIDRDKQIGAAHLQKQAAQLGHEQVNEHITTTMHTAYGEMVLAQKTHALLQQNRQAAKENLRIQ
TLSFQEGFGTVAGVVDAQSALSQIDSETALNAYRYLLALATLLHYTGTIDDFDDYLSLDDAHKL

## Claims

1. An immunogenic fragment of an isolated polypeptide comprising an amino acid sequence which has at least 85% identity to the amino acid sequence of: SEQ ID NO:2 over the entire length of SEQ ID NO:2 in which the immunogenic fragment comprises more than 15 contiguous amino acids from the amino acid sequence of SEQ ID NO:2 and which is capable of raising an immune response if necessary, when coupled to a carrier, which recognises the polypeptide of SEQ ID NO:2, and wherein the immunogenic fragment is not the amino acid sequence of SEQ ID NO: 2.

2. The immunogenic fragment as claimed in claim 1 in which the amino acid sequence has at least 95% identity to the amino acid sequence of SEQ ID NO:2, over the entire length of SEQ ID NO:2.

3. The immunogenic fragment as claimed in claim 1 in which the isolated polypeptide comprises the amino acid sequence of SEQ ID NO:2.

4. The immunogenic fragment as claimed in claim 1 in which the isolated polypeptide consists of the sequence of SEQ ID N0:2.

5. A immunogenic fragment as claimed in any of claims 1 to 4 wherein said polypeptide is part of a larger fusion protein.

**6.** An isolated polynucleotide encoding a polypeptide as claimed in any of claims 1 to 5.

**7.** An expression vector comprising an isolated polynucleotide according to claim 6.

**8.** A recombinant live microorganism comprising the expression vector of claim 7.

**9.** A host cell comprising the expression vector of claim 7.

**10.** An isolated subcellular fraction or an isolated membrane of the host cell of claim 9.

**11.** A membrane of a recombinant microorganism which has upregulated expression of an isolated polypeptide comprising an amino acid sequence which has at least 85% identity to the amino acid sequence of: SEQ ID NO:2 over the entire length of SEQ ID N0:2, or the immunogenic fragment as claimed in any one of claims 1 to 5.

**12.** A process for producing the immunogenic fragment of claims 1 to 5 comprising culturing a host cell of claim 9 under conditions sufficient for the production of said polypeptide and recovering the polypeptide from the culture medium.

**13.** A process for expressing a polynucleotide of claim 6 comprising transforming a host cell with an expression vector comprising at least one of said polynucleotides and culturing said host cell under conditions sufficient for expression of any one of said polynucleotides.

**14.** A bacterial outer-membrane vesicle obtained from a host cell comprising a nucleic acid vector comprising a nucleotide sequence encoding a polypeptide that has at least 85% identity to the amino acid sequence of SEQ ID NO:2 over the entire length of SEQ ID NO:2; a nucleotide sequence that has at least 85% identity to a nucleotide sequence encoding a polypeptide of SEQ ID NO:2 over the entire coding region; a nucleotide sequence which has at least 85% identity to that of SEQ ID NO: 1 over the entire length of SEQ ID NO: or a polynucleotide as claimed in claim 6, having a modified upstream region containing a heterologous regulatory element.

**15.** A vaccine composition comprising an effective amount of an isolated polypeptide comprising an amino acid sequence which has at least 85% identity to the amino acid sequence of SEQ ID NO:2 over the entire length of SEQ ID NO: 2, or the immunogenic fragment of any one of claims 1 to 5 and a pharmaceutically acceptable carrier.

**16.** A vaccine composition comprising an effective amount of a polynucleotide comprising a nucleotide sequence encoding a polypeptide that has at least 85% identity to the amino acid sequence of SEQ ID NO:2 over the entire length of SEQ ID NO:2 or a nucleotide sequence complementary to said isolated polynucleotide; a polynucleotide comprising a nucleotide sequence that has at least 85% identity to a nucleotide sequence encoding a polypeptide of SEQ ID NO:2 over the entire coding region or a nucleotide sequence complementary to said isolated polynucleotide; a polynucleotide which comprises a nucleotide sequence which has at least 85% identity to that of SEQ ID NO:1 over the entire length of SEQ ID NO:1 or a nucleotide sequence complementary to said isolated polynucleotide; or a polynucleotide as claimed in claim 6 and a pharmaceutically acceptable carrier.

**17.** A vaccine composition comprising an effective amount of a bacterial outer-membrane vesicle of claim 14 and a pharmaceutically accpetable carrier.

**18.** The vaccine composition according to any one of claims 15 to 17 wherein said composition comprises at least one other *Moraxella catarrhalis* antigen.

**19.** An antibody generated against the immunological fragment as claimed in any one of claims 1 to 5.

**20.** A method of diagnosing a *Moraxella catarrhalis* infection, comprising identifying an immunogenic fragment as claimed in any one of claims 1 - 5, or an antibody as claimed in claim 19, present within a biological sample from an animal suspected of having such an infection.

**21.** Use of a composition comprising an immunologically effective amount of an isolated polypeptide comprising an amino acid sequence which has at least 85% identity to the amino acid sequence of: SEQ ID NO:2 over the entire length of SEQ ID NO:2, or an immunogenic fragment as claimed in any one of claims 1 - 5 in the preparation of a medicament for use in generating an immune response in an animal.

**22.** Use of a composition comprising an immunologically effective amount of a polynucleotide comprising a nucleotide sequence encoding a polypeptide that has at least 85% identity to the amino acid sequence of SEQ ID NO:2 over the entire length of SEQ ID NO:2 or a nucleotide sequence complementary to said isolated polynucleotide; a polynucleotide comprising a nucleotide sequence that has at least 85% identity to a nucleotide sequence encoding a polypeptide of SEQ ID NO:2 over the entire coding region or a nucleotide sequence complementary to said isolated polynucleotide; a polynucleotide which comprises a nucleotide sequence which has at least 85% identity to that of SEQ ID NO: 1 over the entire length of SEQ ID NO:1 or a nucleotide sequence complementary to said isolated polynucleotide; or a polynucleotide as claimed in claim 6 in the preparation of a medicament for use in generating an immune response in an animal.

**23.** A therapeutic composition useful in treating humans with *Moraxella catarrhalis* disease comprising at least one antibody as claimed in claim 19 and a suitable pharmaceutical carrier.

## Patentansprüche

**1.** Immunogenes Fragment eines isolierten Polypeptids, das eine Aminosäuresequenz umfasst, die wenigstens 85 % Identität mit der Aminosäuresequenz von SEQ ID NO:2 über die gesamte Länge von SEQ ID NO:2 hat, worin das immunogene Fragment mehr als 15 zusammenhängende Aminosäuren aus der Aminosäuresequenz von SEQ ID NO:2 umfasst und eine Immunreaktion hervorrufen kann, nach Bedarf bei Kupplung an einen Träger, die das Polypeptid von SEQ ID N0:2 erkennt, und worin das immunogene Fragment nicht die Aminosäuresequenz von SEQ ID NO:2 ist.

**2.** Immunogenes Fragment gemäss Anspruch 1, worin die Aminosäuresequenz wenigstens 95 % Identität mit der Aminosäuresequenz von SEQ ID NO:2 über die gesamte Länge von SEQ ID NO:2 hat.

**3.** Immunogenes Fragment gemäss Anspruch 1, worin das isolierte Polypeptid die Aminosäuresequenz von SEQ ID NO:2 umfasst.

**4.** Immunogenes Fragment gemäss Anspruch 1, worin das isolierte Polypeptid aus der Sequenz von SEQ ID NO:2 besteht.

**5.** Immunogenes Fragment gemäss einem der Ansprüche 1 bis 4, worin das Polypeptid Teil eines grösseren Fusionsproteins ist.

**6.** Isoliertes Polynukleotid, das ein Polypeptid gemäss einem der Ansprüche 1 bis 5 codiert.

**7.** Expressionsvektor, der ein isoliertes Polynukleotid gemäss Anspruch 6 umfasst.

**8.** Rekombinanter lebender Mikroorganismus, der den Expressionsvektor gemäss Anspruch 7 umfasst.

**9.** Wirtszelle, die den Expressionsvektor gemäss Anspruch 7 umfasst.

**10.** Isolierte subzelluläre Fraktion oder isolierte Membran der Wirtszelle gemäss Anspruch 9.

**11.** Membran eines rekombinanten Mikroorganismus, der eine aufregulierte Expression eines isolierten Polypeptids aufweist, das eine Aminosäuresequenz, die wenigstens 85 % Identität mit der Aminosäuresequenz von SEQ ID N0:2 über die gesamte Länge von SEQ ID NO:2 hat, oder das immunogene Fragment gemäss einem der Ansprüche 1 bis 5 umfasst.

**12.** Verfahren zur Herstellung des immunogenen Fragments gemäss Ansprüchen 1 bis 5, umfassend das Kultivieren einer Wirtszelle gemäss Anspruch 9 unter Bedingungen, die ausreichend zur Erzeugung des Polypeptids sind, und Gewinnen des Polypeptids aus dem Kulturmedium.

**13.** Verfahren zur Expression eines Polynukleotids gemäss Anspruch 6, umfassend das Transformieren einer Wirtszelle mit einem Expressionsvektor, der wenigstens eines der Polynukleotide umfasst, und Kultivieren der Wirtszelle unter Bedingungen, die ausreichend zur Expression eines der Polynukleotide sind.

**14.** Bakterielles Aussenmembranvesikel, das aus einer Wirtszelle erhalten wird, die einen Nukleinsäurevektor umfasst, der eine Nukleotidsequenz, die ein Polypeptid codiert, das wenigstens 85 % Identität mit der Aminosäuresequenz von SEQ ID NO:2 über die gesamte Länge von SEQ ID NO:2 hat; eine Nukleotidsequenz, die wenigstens 85 % Identität mit einer Nukleotidsequenz, die ein Polypeptid von SEQ ID NO:2 codiert, über die gesamte codierende Region hat; eine Nukleotidsequenz, die wenigstens 85 % Identität mit derjenigen von SEQ ID NO:1 über die gesamte Länge von SEQ ID NO:1 hat, oder ein Polynukleotid gemäss Anspruch 6 mit einer modifizierten Stromaufwärtsregion, die ein heterologes regulatorisches Element enthält, umfasst.

**15.** Impfstoffzusammensetzung, die eine wirksame Menge eines isolierten Polypeptids, das eine Aminosäuresequenz umfasst, die wenigstens 85 % Identität mit der Aminosäuresequenz von SEQ ID NO:2 über die gesamte Länge von SEQ ID NO:2 hat, oder des immunogenen Fragments gemäss einem der Ansprüche 1 bis 5 und einen pharmazeutisch akzeptablen Träger umfasst.

**16.** Impfstoffzusammensetzung, die eine wirksame Menge eines Polynukleotids, das eine Nukleotidsequenz, die ein Polypeptid codiert, das wenigstens 85 % Identität mit der Aminosäuresequenz von SEQ ID NO:2 über die gesamte Länge von SEQ ID NO:2 hat, oder eine zu dem isolierten Polynukleotid komplementäre Nukleotidsequenz umfasst; eines Polynukleotids, das eine Nukleotidsequenz, die wenigstens 85 % Identität mit einer Nukleotidsequenz, die ein Polypeptid von SEQ ID NO:2 codiert, über die gesamte codierende Region hat, oder eine zu dem isolierten Polynukleotid komplementäre Nukleotidsequenz umfasst; eines Polynukleotids, das eine Nukleotidsequenz, die wenigstens 85 % Identität zu derjenigen von SEQ ID NO:1 über die gesamte Länge von SEQ ID NO:1 hat, oder eine zu dem isolierten Polynukleotid komplementäre Nukleotidsequenz umfasst; oder eines Polynukleotids gemäss Anspruch 6 und einen pharmazeutisch akzeptablen Träger umfasst.

**17.** Impfstoffzusammensetzung, die eine wirksame Menge eines bakteriellen Aussenmembranvesikels gemäss Anspruch 14 und einen pharmazeutisch akzeptablen Träger umfasst.

**18.** Impfstoffzusammensetzung gemäss einem der Ansprüche 15 bis 17, worin die Zusammensetzung wenigstens ein anderes Moraxella catarrhalis-Antigen umfasst.

**19.** Antikörper, der gegen das immunologische Fragment gemäss einem der Ansprüche 1 bis 5 erzeugt ist.

**20.** Verfahren zum Diagnostizieren einer Moraxella catarrhalis-Infektion, das das Identifizieren eines immunogenen Fragments gemäss einem der Ansprüche 1 bis 5 oder eines Antikörpers gemäss Anspruch 19 umfasst, das/der innerhalb einer biologischen Probe aus einem Tier, das einer solchen Infektion verdächtigt wird, vorhanden ist.

**21.** Verwendung einer Zusammensetzung, die eine immunologisch wirksame Menge eines isolierten Polypeptids, das eine Aminosäuresequenz umfasst, die wenigstens 85 % Identität mit der Aminosäuresequenz von SEQ ID NO:2 über die gesamte Länge von SEQ ID NO:2 hat, oder eines immunogenen Fragments gemäss einem der Ansprüche 1 bis 5 umfasst, in der Herstellung eines Medikaments zur Verwendung in der Erzeugung einer Immunreaktion in einem Tier.

**22.** Verwendung einer Zusammensetzung, die eine immunologisch wirksame Menge eines Polynukleotids, das eine Nukleotidsequenz, die ein Polypeptid codiert, das wenigstens 85 % Identität mit der Aminosäuresequenz von SEQ ID NO:2 über die geamte Länge von SEQ ID NO:2 hat, oder eine zu dem isolierten Polynukleotid komplementäre Nukleotidsequenz umfasst; eines Polynukleotids, das eine Nukleotidsequenz, die wenigstens 85 % Identität mit einer Nukleotidsequenz, die ein Polypeptid von SEQ ID NO:2 codiert, über die gesamte codierende Region hat, oder eine zu dem isolierten Polynukleotid komplementäre Nukleotidsequenz umfasst; eines Polynukleotids, das eine Nukleotidsequenz, die wenigstens 85 % Identität mit derjenigen von SEQ ID NO:1 über die gesamte Länge von SEQ ID NO:1 hat, oder eine zu dem isolierten Polynukleotid komplementäre Nukleotidsequenz umfasst; oder eines Polynukleotids gemäss Anspruch 6 umfasst, in der Herstellung eines Medikaments zur Verwendung in der Erzeugung einer Immunreaktion in einem Tier.

**23.** Therapeutische Zusammensetzung, die nützlich in der Behandlung von Menschen mit Moraxella catarrhalis-Erkrankung ist und die wenigstens einen Antikörper gemäss Anspruch 19 und einen geeigneten pharmazeutischen Träger umfasst.

## Revendications

1. Fragment immunogène d'un polypeptide isolé comprenant une séquence d'acides aminés qui est au moins à 85 % identique à la séquence d'acides aminés de : SEQ ID NO:2 sur la totalité de la longueur de SEQ ID NO:2 dans lequel le fragment immunogène comprend plus de 15 acides aminés contigus provenant de la séquence d'acides aminés de SEQ ID NO:2 et qui est capable de produire une réponse immunitaire si nécessaire, lorsque couplé à un porteur, qui reconnaît le polypeptide de SEQ ID NO:2, et dans lequel le fragment immunogène n'est pas la séquence d'acides aminés de SEQ ID NO:2.

2. Fragment immunogène selon la revendication 1, dans lequel la séquence d'acides aminés est au moins à 95 % identique à la séquence d'acides aminés de SEQ ID NO : 2, sur la totalité de la longueur de SEQ ID NO:2.

3. Fragment immunogène selon la revendication 1, dans lequel le polypeptide isolé comprend la séquence d'acides aminés de SEQ ID NO:2.

4. Fragment immunogène selon la revendication 1, dans lequel le polypeptide isolé est constitué de la séquence de SEQ ID NO:2.

5. Fragment immunogène selon l'une quelconque des revendications 1 à 4, dans lequel ledit polypeptide fait partie d'une protéine hybride plus grande.

6. Polynucléotide isolé codant pour un polypeptide selon l'une quelconque des revendications 1 à 5.

7. Vecteur d'expression comprenant un polynucléotide isolé selon la revendication 6.

8. Micro-organisme vivant recombinant comprenant le vecteur d'expression selon la revendication 7.

9. Cellule hôte comprenant le vecteur d'expression selon la revendication 7.

10. Fraction subcellulaire isolée ou membrane isolée de la cellule hôte selon la revendication 9.

11. Membrane d'un micro-organisme recombinant qui a une expression régulée à la hausse d'un polypeptide isolé comprenant une séquence d'acides aminés qui est au moins à 85 % identique à la séquence d'acides aminés de : SEQ ID NO:2 sur la totalité de la longueur de SEQ ID NO:2, ou le fragment immunogène selon l'une quelconque des revendications 1 à 5.

12. Procédé de production du fragment immunogène selon les revendications 1 à 5, comprenant la mise en culture d'une cellule hôte selon la revendication 9 dans des conditions suffisantes pour la production dudit polypeptide et la récupération du polypeptide dans le milieu de culture.

13. Procédé d'expression d'un polynucléotide selon la revendication 6, comprenant la transformation d'une cellule hôte avec un vecteur d'expression comprenant au moins un desdits polynucléotides et la mise en culture de ladite cellule hôte dans des conditions suffisantes pour l'expression de l'un quelconque desdits polynucléotides.

14. Vésicule de membrane externe bactérienne obtenue à partir d'une cellule hôte comprenant un vecteur d'acide nucléique comprenant une séquence nucléotidique codant pour un polypeptide qui est au moins à 85 % identique à la séquence d'acides aminés de SEQ ID N0:2 sur la totalité de la longueur de SEQ ID NO:2 ; une séquence nucléotidique qui est au moins à 85 % identique à une séquence nucléotidique codant pour un polypeptide de SEQ ID NO:2 sur la totalité de la région codante ; une séquence nucléotidique qui est au moins à 85 % identique à celle de SEQ ID NO:1 sur la totalité de la longueur de SEQ ID NO:1 ou un polynucléotide selon la revendication 6, ayant une région en amont modifiée contenant un élément régulateur hétérologue.

15. Composition de vaccin comprenant une quantité efficace d'un polypeptide isolé comprenant une séquence d'acides aminés qui est à 85 % au moins identique à la séquence d'acides aminés de: SEQ ID NO:2 sur la totalité de la longueur de SEQ ID NO:2, ou le fragment immunogène selon l'une quelconque des revendications 1 à 5 et un porteur pharmaceutiquement acceptable.

16. Composition de vaccin comprenant une quantité efficace d'un polynucléotide comprenant une séquence nucléotidique codant pour un polypeptide qui est au moins à 85 % identique à la séquence d'acides aminés de SEQ ID NO: 2 sur la totalité de la longueur de SEQ ID NO:2 ou une séquence nucléotidique complémentaire dudit polynucléotide isolé ; un polynucléotide comprenant une séquence nucléotidique qui est au moins à 85 % identique à la séquence nucléotidique codant pour un polypeptide de SEQ ID NO:2 sur la totalité de la région codante ou une séquence nucléotidique complémentaire dudit polynucléotide isolé ; un polynucléotide qui comprend une séquence nucléotidique qui est au moins à 85 % identique à celle de SEQ ID NO:1 sur le totalité de la longueur de SEQ ID NO: 1 ou une séquence nucléotidique complémentaire dudit polynucléotide isolé ; ou un polynucléotide selon la revendication 6 et un porteur pharmaceutiquement acceptable.

**17.** Composition de vaccin comprenant une quantité efficace d'une vésicule de membrane externe bactérienne selon la revendication 14 et un porteur pharmaceutiquement acceptable.

**18.** Composition de vaccin selon l'une quelconque des revendications 15 à 17, dans laquelle ladite composition comprend au moins un autre antigène *Moraxella catarrhalis.*

**19.** Anticorps généré contre le fragment immunologique selon l'une quelconque des revendications 1 à 5.

**20.** Méthode de diagnostic d'une infection *Moraxella catarrhalis,* comprenant l'identification d'un fragment immunogène selon l'une quelconque des revendications 1 à 5, ou un anticorps selon la revendication 19, présent dans un échantillon biologique d'un animal soupçonné d'avoir une telle infection.

**21.** Utilisation d'une composition comprenant une quantité immunologiquement efficace d'un polypeptide isolé comprenant une séquence d'acides aminés qui est au moins à 85 % identique à la séquence d'acides aminés de : SEQ ID NO:2 sur la totalité de la longueur de SEQ ID NO:2, ou un fragment immunogène selon l'une quelconque des revendications 1 à 5 dans la préparation d'un médicament pour une utilisation visant à générer une réponse immunitaire chez un animal.

**22.** Utilisation d'une composition comprenant une quantité immunologiquement efficace d'un polynucléotide comprenant une séquence nucléotidique codant pour un polypeptide qui est au moins à 85 % identique à la séquence d'acides aminés de SEQ ID NO:2 sur la totalité de la longueur de SEQ ID N0:2 ou une séquence nucléotidique complémentaire dudit polynucléotide isolé ; un polynucléotide comprenant une séquence nucléotidique qui est au moins à 85 % identique à la séquence nucléotidique codant pour un polypeptide de SEQ ID NO:2 sur la totalité de la région codante ou une séquence nucléotidique complémentaire dudit polynucléotide isolé ; un polynucléotide qui comprend une séquence nucléotidique qui est au moins à 85 % identique à celle de SEQ ID NO:1 sur la totalité de la longueur de SEQ ID NO:1 ou une séquence nucléotidique complémentaire dudit polynucléotide isolé ; ou un polynucléotide selon la revendication 6 dans la préparation d'un médicament pour une utilisation visant à générer une réponse immunitaire chez un animal.

**23.** Composition thérapeutique utile pour le traitement chez les humains de la maladie *Moraxella catarrhalis* comprenant au moins un anticorps selon la revendication 19 et un porteur adapté pharmaceutiquement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0078968 A
- WO 9429458 A
- WO 9422914 A
- US 4946778 A
- EP 0552267 B1
- WO 9400153 A
- WO 9517209 A
- GB 2220211 A
- EP 0689454 B1
- EP 0689454 A
- US 5057540 A
- WO 9633739 A
- WO 9602555 A
- WO 9517210 A


### Non-patent literature cited in the description


- **KLEIN, JO.** *Clin.Inf.Dis,* 1994, vol. 19, 823
- **MURPHY, TF.** *Microbiol.Rev.,* 1996, vol. 60, 267
- **DICKINSON, DP et al.** *J. Infect.Dis.,* 1988, vol. 158, 205
- **FADEN, HL et al.** *Ann.Otorhinol.Laryngol,* 1991, vol. 100, 612
- **FADEN, HL et al.** *J. Infect.Dis.,* 1994, vol. 169, 1312
- **CHAPMAN, AJ et al.** *J. Infect.Dis,* 1985, vol. 151, 878
- **HOL, C et al.** *Lancet,* 1993, vol. 341, 1281
- **JORDAN, KL et al.** *Am.J.Med.,* 1990, vol. 88 (5A), 28S
- **SETHI, S et al.** *Infect.Immun.,* 1995, vol. 63, 1516
- **CHEN D. et al.** *Infect.Immun.,* 1999, vol. 67, 1310
- *Gene,* 1986, vol. 43, 265-272
- *Biotechnology,* 1992, vol. 10, 795-798
- **MANIATIS, T. ; FRITSCH, E.F. ; SAMBROOK et al.** MOLECULAR CLONING, A LABORATORY MANUAL. Cold Spring Harbor Laboratory Press, 1989
- **GENTZ et al.** *Proc. Natl. Acad. Sci.,* 1989, vol. 86, 821-824
- **WILSON et al.** *Cell,* 1984, vol. 37, 767
- **SAMBROOK et al.** Molecular Cloning: A Laboratory Manual. Cold Spring Harbor, 1989
- **FROHMAN et al.** *PNAS USA,* 1988, vol. 85, 8998-9002
- **WOLFF et al.** *Hum Mol Genet,* 1992, vol. 1, 363
- **MANTHORPE et al.** *Hum. Gene Ther.,* 1983, vol. 4, 419
- **WU et al.** *J Biol Chem.,* 1989, vol. 264, 16985
- **BENVENISTY ; RESHEF.** *PNAS USA,* 1986, vol. 83, 9551
- **KANEDA et al.** *Science,* 1989, vol. 243, 375
- **TANG et al.** *Nature,* 1992, vol. 356, 152
- **EISENBRAUN et al.** *DNA Cell Biol,* 1993, vol. 12, 791
- **SEEGER et al.** *PNAS USA,* 1984, vol. 81, 5849
- **DAVIS et al.** *BASIC METHODS IN MOLECULAR BIOLOGY,* 1986
- **SAMBROOK et al.** MOLECULAR CLONING: A LABORATORY MANUAL. Cold Spring Harbor Laboratory Press, 1989
- **MYERS et al.** *Science,* 1985, vol. 230, 1242
- **COTTON et al.** *Proc. Natl. Acad Sci.,* 1985, vol. 85, 4397-4401
- **CHEE et al.** *Science,* 1996, vol. 274, 610
- **KOHLER, G. ; MILSTEIN, C.** *Nature,* 1975, vol. 256, 495-497
- **KOZBOR et al.** *Immunology Today,* 1983, vol. 4, 72
- **COLE et al.** MONOCLONAL ANTIBODIES AND CANCER THERAPY. Alan R. Liss, Inc, 1985, 77-96
- **MCCAFFERTY et al.** *Nature,* 1990, vol. 348, 552-554
- **MARKS et al.** *Biotechnology,* 1992, vol. 10, 779-783
- **CLACKSON et al.** *Nature,* 1991, vol. 352, 628
- **JONES et al.** *Nature,* 1986, vol. 321, 522-525
- **TEMPEST et al.** *Biotechnology,* 1991, vol. 9, 266-273
- **COLIGAN et al.** Current Protocols in Immunology. 1991, vol. 1
- **D. BENNETT et al.** *J Mol Recognition,* 1995, vol. 8, 52-58
- **K. JOHANSON et al.** *J Biol Chem,* 1995, vol. 270 (16), 9459-9471
- **OKANO.** *J. Neurochem.,* 1991, vol. 56, 560

- OLIGODEOXYNUCLEOTIDES AS ANTISENSE INHIBITORS OF GENE EXPRESSION. CRC Press, 1988
- **ZHOU, L et al.** *FEMS Microbiol. Lett.,* 1998, vol. 163, 223-228
- **MOSMANN, T.R. ; COFFMAN, R.L.** TH1 and TH2 cells: different patterns of lymphokine secretion lead to different functional properties. *Annual Review of Immunology,* 1989, vol. 7, 145-173
- Computational Molecular Biology. Oxford University Press, 1988
- Biocomputing: Informatics and Genome Projects. Academic Press, 1993
- Computer Analysis of Sequence Data. Humana Press, 1994, vol. I
- **HEINE, G.** Sequence Analysis in Molecular Biology. Academic Press, 1987
- Sequence Analysis Primer. M Stockton Press, 1991
- **CARILLO, H. ; LIPMAN, D. ; SIAM J.** *Applied Math.,* 1988, vol. 48, 1073
- **DEVEREUX, J. et al.** *Nucleic Acids Research,* 1984, vol. 12 (1), 387
- **ALTSCHUL, S.F. et al.** *J. Molec. Biol.,* 1990, vol. 215, 403-410
- **PEARSON ; LIPMAN.** *Proc. Natl. Acad. Sci. USA,* 1988, vol. 85, 2444-2448
- **ALTSCHUL, S. et al.** BLAST Manual. *NCBI NLM NIH Bethesda, MD 20894*
- **ALTSCHUL, S. et al.** *J. Mol. Biol.,* 1990, vol. 215, 403-410
- **NEEDLEMAN ; WUNSCH.** *J. Mol Biol.,* 1970, vol. 48, 443-453
- **HENIKOFF ; HENIKOFF.** *Proc. Natl. Acad. Sci. USA.,* 1992, vol. 89, 10915-10919
- *Antimicrob. Agents Chemother.,* 1982, vol. 21, 506-508

SEQUENCE LISTING

<110> SmithKline Beecham Biologicals S.A.
<120> Novel Compounds
<130> BM45414
<160> 6
<170> FastSEQ for Windows Version 3.0
<210> 1
<211> 1035
<212> DNA
<213> *Moraxella catarrhalis*
<400> 1

```
atgaaagcac aatttacaac cctaaaagtc atcggcggac tatttgtagg tggcatgatg      60
ctaaatgcac atgcagcacc tgaattatat ggttacgcaa atcttgtcgt ttctgctaat     120
cataaaaaaa caaccaacaa gacaaccaat gtcagtacat caacaacaga ccgcccctat     180
ctgtatagca gcggatcacg cattggcctt agagggtctg aaaagttaaa tgatgattat     240
gaggttttgt acaagttgga gtaccgtcta gaaaatgatg gtgatttacg caatgaaaaa     300
atcaagcagc cagatggtac tgaaaagact gttgctaaaa cacgcaattt tgaggctcgt     360
gattcatgga ttggcgtgaa gcataaaaag tatggtacca tcaaggcggg tcgtatgttg     420
tctttggatc catatgtgcg ttatactgcc tatttggcgt caggcgtaga tggggtgcgt     480
accaataata ccattgcata cgaatcacca aaaatcaaag atgttagttt tcaggcgatg     540
tacatcttag atgaaaataa agagacagat accattgatc gtgatggtta ttcattgctt     600
gtcaagaaaa acacagatac atataatgtt ggtgcagctt atgcctattt tggtaaagca     660
aaaacctctt atggcaagat taactatact gcgcgtgtga caggtaatta taaaattaat     720
gaggattata aagtaggtgg tatctatcag catgtcggct atgccaatga cgacagtgcc     780
aaaaataata cagaacaagc tgttggcgtg gctttgcagc attttaaaga caaatggact     840
cataccgcac acatgaatct tgtgaataat cctagtggga aaaagggcga tgggtttgag     900
ctgatcggag caattgaccg tgatatttct aaaaatgtct ctgcaggcat ggatatcacc     960
tatggtaact ttaactacgc aacagaaaaa gaatcttaca tcaacccaac tatctatgcg    1020
actgtatatt tttaa                                                     1035
```

<210> 2
<211> 344
<212> PRT
<213> *Moraxella catarrhalis*
<400>2

```
Met Lys Ala Gln Phe Thr Thr Leu Lys Val Ile Gly Gly Leu Phe Val
 1               5                   10                  15
Gly Gly Met Met Leu Asn Ala His Ala Ala Pro Glu Leu Tyr Gly Tyr
            20                  25                  30
Ala Asn Leu Val Val Ser Ala Asn His Lys Lys Thr Thr Asn Lys Thr
        35                  40                  45
Thr Asn Val Ser Thr Ser Thr Thr Asp Arg Pro Tyr Leu Tyr Ser Ser
    50                  55                  60
Gly Ser Arg Ile Gly Leu Arg Gly Ser Glu Lys Leu Asn Asp Asp Tyr
65                  70                  75                  80
Glu Val Leu Tyr Lys Leu Glu Tyr Arg Leu Glu Asn Asp Gly Asp Leu
```

```
                        85                  90                  95
Arg Asn Glu Lys Ile Lys Gln Pro Asp Gly Thr Glu Lys Thr Val Ala
            100                 105                 110
Lys Thr Arg Asn Phe Glu Ala Arg Asp Ser Trp Ile Gly Val Lys His
        115                 120                 125
Lys Lys Tyr Gly Thr Ile Lys Ala Gly Arg Met Leu Ser Leu Asp Pro
    130                 135                 140
Tyr Val Arg Tyr Thr Ala Tyr Leu Ala Ser Gly Val Asp Gly Val Arg
145                 150                 155                 160
Thr Asn Asn Thr Ile Ala Tyr Glu Ser Pro Lys Ile Lys Asp Val Ser
                165                 170                 175
Phe Gln Ala Met Tyr Ile Leu Asp Glu Asn Lys Glu Thr Asp Thr Ile
            180                 185                 190
Asp Arg Asp Gly Tyr Ser Leu Leu Val Lys Lys Asn Thr Asp Thr Tyr
        195                 200                 205
Asn Val Gly Ala Ala Tyr Ala Tyr Phe Gly Lys Ala Lys Thr Ser Tyr
    210                 215                 220
Gly Lys Ile Asn Tyr Thr Ala Arg Val Thr Gly Asn Tyr Lys Ile Asn
225                 230                 235                 240
Glu Asp Tyr Lys Val Gly Gly Ile Tyr Gln His Val Gly Tyr Ala Asn
                245                 250                 255
Asp Asp Ser Ala Lys Asn Asn Thr Glu Gln Ala Val Gly Val Ala Leu
            260                 265                 270
Gln His Phe Lys Asp Lys Trp Thr His Thr Ala His Met Asn Leu Val
        275                 280                 285
Asn Asn Pro Ser Gly Lys Lys Gly Asp Gly Phe Glu Leu Ile Gly Ala
    290                 295                 300
Ile Asp Arg Asp Ile Ser Lys Asn Val Ser Ala Gly Met Asp Ile Thr
305                 310                 315                 320
Tyr Gly Asn Phe Asn Tyr Ala Thr Glu Lys Glu Ser Tyr Ile Asn Pro
                325                 330                 335
Thr Ile Tyr Ala Thr Val Tyr Phe
            340
```

<210> 3
<211> 2037
<212> DNA
<213> *Moraxella catarrhalis*
<400> 3

```
atgcgtttta taccttttgc tgtaactctt ttatctttta ctggctcctc catcttttct      60
gtggccaaag ctcagattgc accagaccca accaaagcaa atctttctgc aataaccgat     120
gatcgtcgtc aggcggctct tgcacattta gcaagacaag atttggcagc aacagatgac     180
gacatgggtg tcgatatcaa tcaaaccaat atcaaccagc caaattggca agatgacaag     240
aatggcgatt catgtttttt ggttgatgag attgactttg tgattgaggg tggagaagat     300
aaacatattc taggtatgac gccgagcaat ttacgcagct tgctatcgcc cctgttggac     360
cggacaaagc caacatatgc tcttaatcac tgcatcaaca atcacaacct atctttaatt     420
gtagatattg cccataatga attactaaag cgtggttatt taactagcag tattagcatt     480
gaagagcagg atttatcaac taaaaaactc actttaacag tccatgctgg caaggtgaaa     540
aaggtgatat tggccgatgc tagcaaaacg ccaacttatg tcaaagccgc cattcctctt     600
aagttcaatc aagcctttcg tctgtcatat ttagaacaag gtctagataa tttaaaacgc     660
atcgatccaa cggcaaccgt gcagattata ccaagcagct ccagtaatgt cgccaatgat     720
attgacatta acaccacaaa ccttggtttt agtgatttac tcattaggat ggaccgcagt     780
caaagcgctg tatttggcat taatattgat aacagtttat ccaaagatta tggcaattat     840
ctgatttctg ctaatgttcg tgctaacaac ctcatgcatc ttaacgatga atggaatctt     900
tctgccaact atccattggc gcgcctgatt gacgctgccc aaaatgatct tggggttcag     960
```

```
gtgggcaagg ataggcaggt taattatcac gcttccttga ctattcctta tggattgtat    1020
aaatttctg ccacacacag ccaccaccaa taccagcaat ttcttgaagg gcttcatgcg    1080
cccttgactt atcatggcac cagcaagaca agctcaattg gattgtctcg tttattgcac    1140
cgtgatggca atcaaaagac tgaaggttat ataaaggtta atcacaaacg aagcagtaat    1200
tatattgatg atgttaatct tgaagtgcaa aaccgccgca caacaggata taacattggc    1260
atcactcatc agcatcatct tcaccaaggt ggttatttgt atgccaactt ggattataag    1320
caaggaacag gggcactaaa agcaaagcct gcacctgaag agcatattta tgatgctttt    1380
gggcgacaac tacccagtga gggttttgca aaagcaccaa tttggtcact atacaccagt    1440
ttccaaaagc catttgtgtt aaacaaccca aatgatactg aacaagcgca agataaagca    1500
acagcaaata aacccattta cacatccatt cctctcactt atactgctag attacaagcc    1560
caatatgcca aacaacttcc tgtaccatct gatttattct atttgggtgg cagatatagc    1620
attaaaggca ttaaagaggg aaattattta tcaggagaac atggctttag cctatcccaa    1680
gaacttgctt ggcaattgcc attacagaat cttaatcagc actttagtac aaatgcaaac    1740
agcgcccagt tgtatgcaag tattgatcaa ggctatgcct atggaaaaaa cactcttaat    1800
aatcaacgcc atatcttggc tggagcggtt ggtatgaggt attattttca aggcagtcaa    1860
gatccaagaa ttcaagaaac acaaaatggc ttgactcatt tcaaagaatc aaatacttat    1920
ctaaataaca caccaaccac agctcatttg gatatattca ttggaaaagg aattaagacg    1980
cctgaattta tgaagaaaga aaccgtcgtg ggtgtgagtg ccagcattga gttttaa       2037
```

<210> 4
<211> 678
<212> PRT
<213> *Moraxella catarrhalis*
<400> 4

```
Met Arg Phe Ile Pro Phe Ala Val Thr Leu Leu Ser Phe Thr Gly Ser
1               5                   10                  15
Ser Ile Phe Ser Val Ala Lys Ala Gln Ile Ala Pro Asp Pro Thr Lys
            20                  25                  30
Ala Asn Leu Ser Ala Ile Thr Asp Asp Arg Arg Gln Ala Ala Leu Ala
        35                  40                  45
His Leu Ala Arg Gln Asp Leu Ala Ala Thr Asp Asp Asp Met Gly Val
    50                  55                  60
Asp Ile Asn Gln Thr Asn Ile Asn Gln Pro Asn Trp Gln Asp Asp Lys
65                  70                  75                  80
Asn Gly Asp Ser Cys Phe Leu Val Asp Glu Ile Asp Phe Val Ile Glu
                85                  90                  95
Gly Gly Glu Asp Lys His Ile Leu Gly Met Thr Pro Ser Asn Leu Arg
            100                 105                 110
Ser Leu Leu Ser Pro Leu Leu Asp Arg Thr Lys Pro Thr Tyr Ala Leu
        115                 120                 125
Asn His Cys Ile Asn Asn His Asn Leu Ser Leu Ile Val Asp Ile Ala
    130                 135                 140
His Asn Glu Leu Leu Lys Arg Gly Tyr Leu Thr Ser Ser Ile Ser Ile
145                 150                 155                 160
Glu Glu Gln Asp Leu Ser Thr Lys Lys Leu Thr Leu Thr Val His Ala
                165                 170                 175
Gly Lys Val Lys Lys Val Ile Leu Ala Asp Ala Ser Lys Thr Pro Thr
            180                 185                 190
Tyr Val Lys Ala Ala Ile Pro Leu Lys Phe Asn Gln Ala Phe Arg Leu
        195                 200                 205
Ser Tyr Leu Glu Gln Gly Leu Asp Asn Leu Lys Arg Ile Asp Pro Thr
    210                 215                 220
Ala Thr Val Gln Ile Ile Pro Ser Ser Ser Ser Asn Val Ala Asn Asp
225                 230                 235                 240
Ile Asp Ile Asn Thr Thr Asn Leu Gly Phe Ser Asp Leu Leu Ile Arg
                245                 250                 255
Met Asp Arg Ser Gln Ser Ala Val Phe Gly Ile Asn Ile Asp Asn Ser
```

```
                260                 265                 270
Leu Ser Lys Asp Tyr Gly Asn Tyr Leu Ile Ser Ala Asn Val Arg Ala
        275                 280                 285
Asn Asn Leu Met His Leu Asn Asp Glu Trp Asn Leu Ser Ala Asn Tyr
    290                 295                 300
Pro Leu Ala Arg Leu Ile Asp Ala Ala Gln Asn Asp Leu Gly Val Gln
305                 310                 315                 320
Val Gly Lys Asp Arg Gln Val Asn Tyr His Ala Ser Leu Thr Ile Pro
                325                 330                 335
Tyr Gly Leu Tyr Lys Phe Ser Ala Thr His Ser His His Gln Tyr Gln
            340                 345                 350
Gln Phe Leu Glu Gly Leu His Ala Pro Leu Thr Tyr His Gly Thr Ser
        355                 360                 365
Lys Thr Ser Ser Ile Gly Leu Ser Arg Leu Leu His Arg Asp Gly Asn
    370                 375                 380
Gln Lys Thr Glu Gly Tyr Ile Lys Val Asn His Lys Arg Ser Ser Asn
385                 390                 395                 400
Tyr Ile Asp Asp Val Asn Leu Glu Val Gln Asn Arg Arg Thr Thr Gly
                405                 410                 415
Tyr Asn Ile Gly Ile Thr His Gln His His Leu His Gln Gly Gly Tyr
            420                 425                 430
Leu Tyr Ala Asn Leu Asp Tyr Lys Gln Gly Thr Gly Ala Leu Lys Ala
        435                 440                 445
Lys Pro Ala Pro Glu Glu His Ile Tyr Asp Ala Phe Gly Arg Gln Leu
    450                 455                 460
Pro Ser Glu Gly Phe Ala Lys Ala Pro Ile Trp Ser Leu Tyr Thr Ser
465                 470                 475                 480
Phe Gln Lys Pro Phe Val Leu Asn Asn Pro Asn Asp Thr Glu Gln Ala
                485                 490                 495
Gln Asp Lys Ala Thr Ala Asn Lys Pro Ile Tyr Thr Ser Ile Pro Leu
            500                 505                 510
Thr Tyr Thr Ala Arg Leu Gln Ala Gln Tyr Ala Lys Gln Leu Pro Val
        515                 520                 525
Pro Ser Asp Leu Phe Tyr Leu Gly Gly Arg Tyr Ser Ile Lys Gly Ile
    530                 535                 540
Lys Glu Gly Asn Tyr Leu Ser Gly Glu His Gly Phe Ser Leu Ser Gln
545                 550                 555                 560
Glu Leu Ala Trp Gln Leu Pro Leu Gln Asn Leu Asn Gln His Phe Ser
                565                 570                 575
Thr Asn Ala Asn Ser Ala Gln Leu Tyr Ala Ser Ile Asp Gln Gly Tyr
            580                 585                 590
Ala Tyr Gly Lys Asn Thr Leu Asn Asn Gln Arg His Ile Leu Ala Gly
        595                 600                 605
Ala Val Gly Met Arg Tyr Tyr Phe Gln Gly Ser Gln Asp Pro Arg Ile
    610                 615                 620
Gln Glu Thr Gln Asn Gly Leu Thr His Phe Lys Glu Ser Asn Thr Tyr
625                 630                 635                 640
Leu Asn Asn Thr Pro Thr Thr Ala His Leu Asp Ile Phe Ile Gly Lys
                645                 650                 655
Gly Ile Lys Thr Pro Glu Phe Met Lys Lys Glu Thr Val Val Gly Val
            660                 665                 670
Ser Ala Ser Ile Glu Phe
        675
```

<210> 5
<211> 1410
<212> DNA
<213> *Moraxella catarrhalis*
<400> 5

atgaaaatgc cctatttttt gcttttggga ttattgttag tacagcaggc tgatgctgat 60
tatagcttgc ttgatgccca aaaacagctc atgagcagca gccctgccat ccaagcaagt 120
catgccttgt atcaagctga tgtcttacaa gcagaatcat tacaaaaatt acactatcct 180
aggatatcgc tcaatgcaca cgcctttgta ctgcaacaaa acagcagcat acccttagat 240
catatcaaag agcagaccgc ccagcacatt aacacccatt ttgaccatcg ctttggcagt 300
gcccccgatg gtctgttgga tgctttgcac aacagcaccc aaaccgccct tgaccgcctg 360
cctgaccatc aagatgtcaa gttacgccat gatggcatta ccccaaacat taccgccacg 420
atacccatct atacaggtgg cttaatcagc agcaccaaaa atatcgccaa tctacaagca 480
cagcgtggta aatttggatt acaagaacgc atctctttgg caaaacttaa tttgattcgc 540
cattatttta atgtgcaatt acaaaagcag ctgaccgaca cacaacaaaa catgcttagt 600
gccatgcagt tacatgtaga taatgcttat aaattagaac agcaaggttt tatcagtcgt 660
ggacagcgta tgcaatttga agtggcacgc aatcaggtac aaagactgta tcaaagcacc 720
caaaatcagc accaaaacag catttatgaa cttgctgttt tgcttggttt gccccacatt 780
gaaccgctaa gcacaccgct gtttatcaac acccagcatc gccccaattg gcaagcatta 840
ctcaaagact cccaaaatac accattaaat caaaagctaa aaaccgacat tttgcttgcc 900
gatgaaaata tcgccctaag acaatccacc aaaaaaccaa aaattgccgc tgtggctcgt 960
tataccttag atgataagcc tgattggttt gcaggtgtgg cggtttctta caacctattc 1020
ttgggcattg accgtgataa gcagattggg gcagcacacc tacaaaaaca agcagcccaa 1080
ttgggtcatg aacaagtcaa tgaacacatc acaaccacca tgcacaccgc ttatggcgag 1140
atggtgctgg cacaaaaaac ccacgcccta ctacaacaaa atcgtcaagc cgccaaagaa 1200
aatttgcgta tccagacgct gtcttttcaa gaagggtttg ggacggtggc aggtgtggtt 1260
gatgcccaaa gtgctttatc acagattgac agcgaaaccg ctctaaatgc gtatcgctac 1320
cttttggcac tggcgacgct gttgcattac acagggacaa ttgatgactt tgatgattat 1380
ttgtctttgg atgatgctca taaactgtga 1410

<210> 6
<211> 469
<212> PRT
<213> *Moraxella catarrhalis*
<400>6

Met Lys Met Pro Tyr Phe Leu Leu Leu Gly Leu Leu Leu Val Gln Gln
1 5 10 15
Ala Asp Ala Asp Tyr Ser Leu Leu Asp Ala Gln Lys Gln Leu Met Ser
20 25 30
Ser Ser Pro Ala Ile Gln Ala Ser His Ala Leu Tyr Gln Ala Asp Val
35 40 45
Leu Gln Ala Glu Ser Leu Gln Lys Leu His Tyr Pro Arg Ile Ser Leu
50 55 60
Asn Ala His Ala Phe Val Leu Gln Gln Asn Ser Ser Ile Pro Leu Asp
65 70 75 80
His Ile Lys Glu Gln Thr Ala Gln His Ile Asn Thr His Phe Asp His
85 90 95
Arg Phe Gly Ser Ala Pro Asp Gly Leu Leu Asp Ala Leu His Asn Ser
100 105 110
Thr Gln Thr Ala Leu Asp Arg Leu Pro Asp His Gln Asp Val Lys Leu
115 120 125
Arg His Asp Gly Ile Thr Pro Asn Ile Thr Ala Thr Ile Pro Ile Tyr
130 135 140
Thr Gly Gly Leu Ile Ser Ser Thr Lys Asn Ile Ala Asn Leu Gln Ala
145 150 155 160
Gln Arg Gly Lys Phe Gly Leu Gln Glu Arg Ile Ser Leu Ala Lys Leu

```
                    165                 170                 175
Asn Leu Ile Arg His Tyr Phe Asn Val Gln Leu Gln Lys Gln Leu Thr
                180                 185                 190
Asp Thr Gln Gln Asn Met Leu Ser Ala Met Gln Leu His Val Asp Asn
            195                 200                 205
Ala Tyr Lys Leu Glu Gln Gln Gly Phe Ile Ser Arg Gly Gln Arg Met
    210                 215                 220
Gln Phe Glu Val Ala Arg Asn Gln Val Gln Arg Leu Tyr Gln Ser Thr
225                 230                 235                 240
Gln Asn Gln His Gln Asn Ser Ile Tyr Glu Leu Ala Val Leu Leu Gly
                245                 250                 255
Leu Pro His Ile Glu Pro Leu Ser Thr Pro Leu Phe Ile Asn Thr Gln
            260                 265                 270
His Arg Pro Asn Trp Gln Ala Leu Leu Lys Asp Ser Gln Asn Thr Pro
        275                 280                 285
Leu Asn Gln Lys Leu Lys Thr Asp Ile Leu Leu Ala Asp Glu Asn Ile
    290                 295                 300
Ala Leu Arg Gln Ser Thr Lys Lys Pro Lys Ile Ala Ala Val Ala Arg
305                 310                 315                 320
Tyr Thr Leu Asp Asp Lys Pro Asp Trp Phe Ala Gly Val Ala Val Ser
                325                 330                 335
Tyr Asn Leu Phe Leu Gly Ile Asp Arg Asp Lys Gln Ile Gly Ala Ala
            340                 345                 350
His Leu Gln Lys Gln Ala Ala Gln Leu Gly His Glu Gln Val Asn Glu
        355                 360                 365
His Ile Thr Thr Thr Met His Thr Ala Tyr Gly Glu Met Val Leu Ala
    370                 375                 380
Gln Lys Thr His Ala Leu Leu Gln Gln Asn Arg Gln Ala Ala Lys Glu
385                 390                 395                 400
Asn Leu Arg Ile Gln Thr Leu Ser Phe Gln Glu Gly Phe Gly Thr Val
                405                 410                 415
Ala Gly Val Val Asp Ala Gln Ser Ala Leu Ser Gln Ile Asp Ser Glu
            420                 425                 430
Thr Ala Leu Asn Ala Tyr Arg Tyr Leu Leu Ala Leu Ala Thr Leu Leu
        435                 440                 445
His Tyr Thr Gly Thr Ile Asp Asp Phe Asp Asp Tyr Leu Ser Leu Asp
    450                 455                 460
Asp Ala His Lys Leu
465
```